# EUROPEAN PATENT SPECIFICATION

(11) **EP 3 017 061 B1**
(45) Date of publication and mention of the grant of the patent: **02.01.2019**
(21) Application number: 14736727.0
(22) Date of filing: 02.07.2014
(51) Int. Cl.: C12Q 1/6886

(54) **MRNA-BASED GENE EXPRESSION FOR PERSONALIZING PATIENT CANCER THERAPY WITH AN MDM2 ANTAGONIST**
MRNA-BASIERTE GENEXPRESSION ZUR PERSONALISIERUNG EINER PATIENTENKREBSTHERAPIE MIT EINEM MDM2-ANTAGONISTEN
EXPRESSION GÉNIQUE À BASE D'ARNM POUR LA PERSONNALISATION DE LA THÉRAPIE ANTICANCÉREUSE D'UN PATIENT PAR UN ANTAGONISTE DE MDM2

(30) Priority: 03.07.2013 US 201361842567 P; 06.12.2013 US 201361912781 P
(43) Date of publication of application: 11.05.2016
(73) Proprietor: F. Hoffmann-La Roche AG, 4070 Basel (CH)
(72) Inventor: CHEN, Gong, Rutherford New Jersey 07070 (US); DANGL, Markus, 82402 Seeshaupt (DE); GEHO, David, Allendale New Jersey 07401 (US); NICHOLS, Gwen, New York New York 10128 (US); ZHONG, Hua, Short Hills New Jersey 07078 (US)
(74) Representative: Beyermann, Jochen Carl
(86) International application number: PCT/EP2014/064039
(87) International publication number: WO 2015/000945

(56) References cited:
- WO-A1-2009/140304
- WO-A2-2004/111603
- US-A1- 2012 046 186
- J. T. PATTON: "Levels of HdmX Expression Dictate the Sensitivity of Normal and Transformed Cells to Nutlin-3", CANCER RESEARCH, vol. 66, no. 6, 15 March 2006 (2006-03-15) , pages 3169-3176, XP055121090, ISSN: 0008-5472, DOI: 10.1158/0008-5472.CAN-05-3832
- B. HU ET AL: "MDMX Overexpression Prevents p53 Activation by the MDM2 Inhibitor Nutlin", JOURNAL OF BIOLOGICAL CHEMISTRY, vol. 281, no. 44, 6 September 2006 (2006-09-06), pages 33030-33035, XP055139882, ISSN: 0021-9258, DOI: 10.1074/jbc.C600147200
- KRAJEWSKI STANISLAW ET AL: "Reduced expression of proapoptotic gene BAX is associated with poor response rates to combination chemotherapy and shorter survival in women with metastatic breast adenocarcinoma", CANCER RESEARCH, AMERICAN ASSOCIATION FOR CANCER RESEARCH, US, vol. 55, no. 19, 1 January 1995 (1995-01-01), pages 4471-4478, XP002161055, ISSN: 0008-5472
- KENSUKE KOJIMA ET AL: "Mdm2 inhibitor Nutlin-3a induces p53-mediated apoptosis by transcription- dependent and transcription-independent mechanisms and may overcome Atm-mediated resistance to fludarabine in chronic lymphocytic leukemia", BLOOD, vol. 108, no. 3, 1 August 2006 (2006-08-01), pages 993-1000, XP055140017, DOI: 10.1182/blood-2005-
- E DRAKOS ET AL: "Activation of the p53 pathway by the MDM2 inhibitor nutlin-3a overcomes BCL2 overexpression in a preclinical model of diffuse large B-cell lymphoma associated with t(14;18)(q32;q21)", LEUKEMIA, vol. 25, no. 5, 11 March 2011 (2011-03-11) , pages 856-867, XP055140019, ISSN: 0887-6924, DOI: 10.1038/leu.2011.28
- C. SADDLER ET AL: "Comprehensive biomarker and genomic analysis identifies p53 status as the major determinant of response to MDM2 inhibitors in chronic lymphocytic leukemia", BLOOD, vol. 111, no. 3, 25 October 2007 (2007-10-25), pages 1584-1593, XP055139885, ISSN: 0006-4971, DOI: 10.1182/blood-2007-09-112698
- BINH VU ET AL: "Discovery of RG7112: A Small-Molecule MDM2 Inhibitor in Clinical Development", ACS MEDICINAL CHEMISTRY LETTERS, vol. 4, no. 5, 9 May 2013 (2013-05-09), pages 466-469, XP055139780, ISSN: 1948-5875, DOI: 10.1021/ml4000657
- SANJEEV SHANGARY ET AL: "Small-Molecule Inhibitors of the MDM2-p53 Protein-Protein Interaction to Reactivate p53 Function: A Novel Approach for Cancer Therapy", ANNUAL REVIEW OF PHARMACOLOGY AND TOXICOLOGY, vol. 49, no. 1, 1 February 2009 (2009-02-01), pages 223-241, XP055075317, ISSN: 0362-1642, DOI: 10.1146/annurev.pharmtox.48.113006.094723 cited in the application
- HEGE O. OHNSTAD ET AL: "Correlation of TP53 and MDM2 genotypes with response to therapy in sarcoma", CANCER, vol. 119, no. 5, 16 November 2012 (2012-11-16), pages 1013-1022, XP055158154, ISSN: 0008-543X, DOI: 10.1002/cncr.27837
- CHRISTOPH R. MÜLLER ET AL: "Potential for treatment of liposarcomas with the MDM2 antagonist Nutlin-3A", INTERNATIONAL JOURNAL OF CANCER, vol. 121, no. 1, 1 July 2007 (2007-07-01), pages 199-205, XP055158062, ISSN: 0020-7136, DOI: 10.1002/ijc.22643

## Description

### BACKGROUND OF THE INVENTION

The TP53 gene encodes a tumor suppressor protein that plays a critical role in the protection during the development of cancers. P53 is crucial in multi-cellular organisms where it regulates cell cycle, and thus acts as a tumor suppressor that is involved in preventing cancer. Further, it is a transcription factor that regulates multiple genes involved in cell cycle control, such as apoptosis, DNA repair and senescence.

Under non-stressed conditions, the level of p53 protein is controlled by MDM2 (Murine Double Minute 2) via a negative feedback loop, wherein MDM2 transcription is driven by p53. MDM2 protein binds to the TP53 protein and blocks its transactivation domain. MDM2 can also function as a p53 ubiquitin ligase, which marks p53 for ubiquitin dependent degradation.

In cells that overexpress MDM2, P53 is inactivated, leading to inefficient growth arrest and apoptosis. Blocking the P53--MDM2 interaction might restore P53 function and could be a novel approach to cancer treatment. Treatment of tumor cells with MDM2 antagonists should enable p53 to mediate its downstream functions, including activation of gene transcription and induction of cell cycle arrest and apoptosis.

TP53 mutations are rare in Acute Myeloid Leukemia (AML) and are generally not considered to be of primary importance in the development of these malignancies. However, MDM2 has been found to be frequently overexpressed in AML, and can enhance the tumorigenic potential and resistance to apoptosis through abrogation of p53 function. It has been found that AML cell lines and 16 primary aAML samples with wild-type p53 responded to MDM2 antagonist (inhibitor) by induction of p53-dependent apoptosis. These findings support the rationale of targeting the p53-MDM2 interaction as a therapeutic strategy for AML.

Based on the proposed mechanism of action of the drug, the presence of functional p53 protein and related pathway effector molecules are required for this class of drugs to be efficacious. Not all patients will have functional p53 proteins and related pathway effector molecules. In order to better determine whether a patient can benefit from therapy, there is a need to discover predictive molecular tests for identifying patients that are most likely to respond to therapy. One approach for assessing potential response to a MDM2 antagonist is to assess whether or not the TP53 gene is mutated. However, this is complicated by the fact that a multitude of mutations can be found in TP53 in cancer. Not all of these mutations will interfere with activity of the p53 protein, further complicating interpretation of TP53 mutational tests. In addition, there is a range of responses to MDM2 antagonists in wild type TP53 cell lines and patients. Therefore, the ability to predict responsiveness to an MDM2 antagonist from an easily interpretable diagnostic tool is an unmet need in clinical development of MDM2 antagonists. To this end, the development of a gene expression signature that reflects p53 pathway activity could provide a means of selecting patients most likely to respond to MDM2 antagonist therapy. Hege O. Ohnstad et al (Cancer, Vol. 119, No. 5; 2012**)** investigate the correlation of TP53 and MDM2 genotypes with response to therapy in sarcoma.

### SUMMARY OF THE INVENTION

In one aspect the present invention relates to a method for predicting the response of a patient having cancer to therapy, wherein the patients therapy comprises the treatment with a compound which acts as inhibitor of the MDM2-p53 interaction, said method comprising the steps of:
a) measuring a level in a sample pre-obtained from the subject to obtain a value or values representing this level; and
b) comparing the value or values from step a) to a standard value or set of standard values.

Compounds which act as inhibitor of the MDM2-p53 interaction (MDM2-inhibitors, MDM2-antagonists) are known in the art. MDM2-inhibitors and methods for making them are for example disclosed in U.S. Patent 8,354,444 B2, as well as in WO 2007/063013, WO 2010/031713 and WO 2011/098398. Additional MDM2-inhibitors are disclosed in WO 2013/135648.
U.S. Patent 8,354,444 B2, as well as WO 2010/031713, WO 2011/098398 disclose for example compounds of formula I, II and IIa 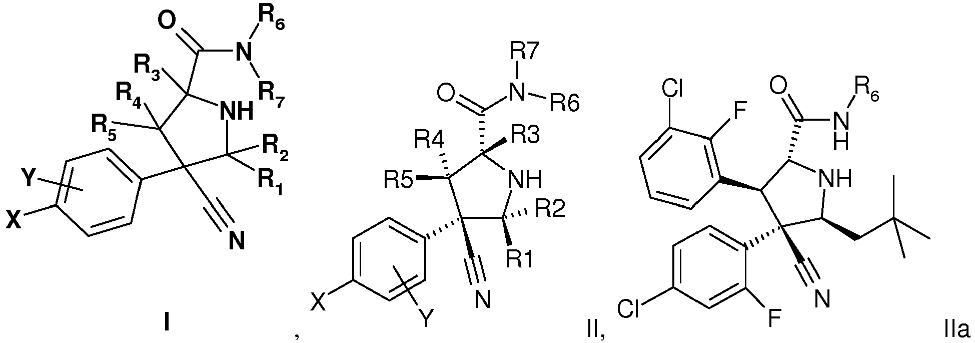 WO 2013/135648 discloses for example compounds of formula III

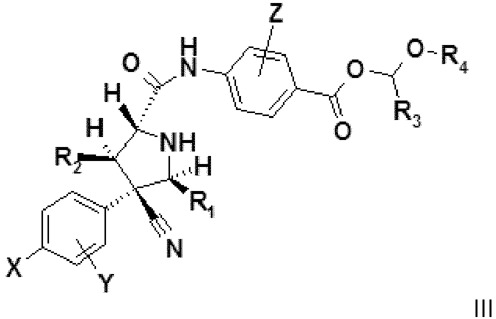

In one aspect, the present invention relates to a method for predicting the response of a patient having cancer to therapy, wherein the patients therapy comprises the treatment with a compound which acts as inhibitor of the MDM2-p53 interaction, preferably an MDM2-inhibitor as specifically disclosed in the working examples of U.S. Patent 8,354,444 B2, or in WO 2007/063013, WO 2010/031713, WO 2011/098398 and WO 2013/135648.
In a further aspect, the disclosure
relates to a method of treating a neoplastic disease, cancer, in a patient in need thereof, comprising measuring a level in a sample from the patient to obtain a value or values representing this level, and treating the subject with a compound of general formula I or a pharmaceutically acceptable derivative thereof as defined above.
A "neoplastic disease" that is potentially treatable based on the MDM2 response is a cancer. In one embodiment, the cancer is selected from the group consisting of breast cancer, prostate cancer, cervical cancer, ovarian cancer, gastric cancer, colorectal cancer (i.e. including colon cancer and rectal cancer), pancreatic cancer, liver cancer, brain cancer, neuroendocrine cancer, lung cancer, kidney cancer, hematological malignancies, melanoma and sarcomas. In another embodiment, the cancer is selected from the group consisting of hematological malignancies, prostate cancer, breast cancer, cervical cancer, ovarian cancer, colorectal cancer, melanoma and lung cancer. In yet another embodiment, the cancer is acute myeloid leukemia (AML), or Myelodysplatic Syndrome (MDS), or Myeloproliferative Neoplams (MPN).

In yet another aspect, disclosed herein is a kit for predicting the response to a compound of formula or a pharmaceutically acceptable derivative thereof, as defined above, comprising reagents necessary for measuring the level in a sample. More preferably the kit also comprises a comparator module which comprises a standard value or set of standard values to which the level of response in the sample is compared. The kit may also comprise a captive reagent.

More preferably the kit comprises an MDM2 inibitor, preferably an MDM2-inhibitor as specifically disclosed in U.S. Patent 8,354,444 B2, or in WO 2007/063013, WO 2010/031713, WO 2011/098398 and WO 2013/135648; or a compound according to formulae IIa or III; or a specific MDM2-inhibitor as disclosed herein.

The present invention identifies a gene expression signature (mRNA) panel based on *in vitro* and clinical data that identify patients most likely to respond to MDM2 antagonist therapy. The mRNA signature is characterized by up-regulations of three genes, specifically MDM2, XPC (xeroderma pigmentosum, complementation group C), BBC3 (BCL2 binding component 3) and down-regulation of CDKN2A (cyclin-dependent kinase inhibitor 2A).

The present invention identifies a 4-gene expression signature (mRNA) for identifying responses to MDM2 antagonist.

The present invention also identifies a 4-gene expression signature (mRNA) for monitoring treatment of cancer in a patient with an MDM2 antagonist.

The baseline expression levels of MDM2, BBC3, CDKN2A, and XPC yield a composite score that discriminates between cell lines and patient-derived clinical specimens that are resistant to therapy, and identifies those that are sensitive (responsive) to the therapy.

As such, the present disclosure relates to a method for identifying sensitivity to MDM2 antagonist therapy. Furthermore, the present disclosure relates to a method for treating a cancer patient with an MDM2 antagonist by testing the sensitivity of the patient before hand by the gene signature (mRNA) panel, more specifically a panel including MDM2, and more specifically a 4-gene expression system.

The present disclosure further provides the predictive signature utilizing mRNA values in determining the effectiveness of MDM2 antagonist therapy to cancers, in particular, AML.

Also disclosed herein is the use of a gene panel containing at least the MDM2 gene as predictive mechanism for determining a patent's response to a disease, particularly cancer, more particularly acute myeloid leukemia (AML), when a patient is to be treated with an MDM2 antagonist.

More particularly, disclosed herein is the use of a four-gene panel in order to determine a patient's response to a disease, particularly AML, when a patient is to be treated with an MDM2 antagonist.

### BRIEF DESCRIPTION OF THE FIGURES

Figure 1: This outline provides the sequence of work that forms the invention.
Figure 2: MDM2 antagonist response mRNA signature *in vitro.*
   Top: The heatmap of the four gene mRNAs (in the original, colored version green corresponds to lower expression, red to higher expression) associated with MDM2 antagonist response for the most sensitive (IC50<=1) and resistant (IC50>=10) cell lines. For the most sensitive cell lines, MDM2, XPC and BBC3 show high expression, and CDKN2A shows low expression. For each cell line TP53 mutation features are at the top of the heatmap (white = wild type). The natural log IC50 values are represented at the bottom.
   Bottom: The boxplot of the 4-gene mRNA signature score for the most sensitive (IC50<=1) and resistant (IC50>=10) cell lines.

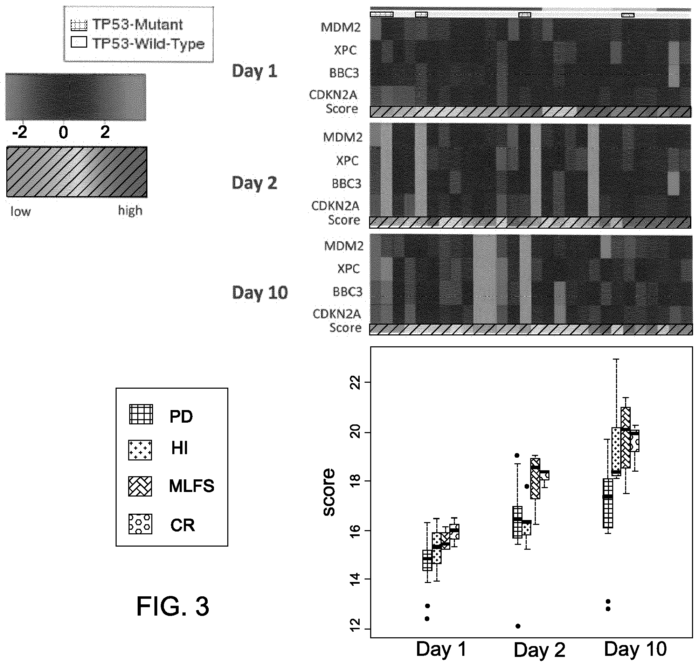

Figure 3: MDM2 antagonist response mRNA signature in AML trial.
   Top: The heatmap of the four gene mRNAs (red in the original colored version corresponds to higher expression) associated with MDM2 antagonist response for the 28 evaluated patients. For each patient TP53 mutation features are at the top of the heatmap (white = wild-type). The clinical efficacy groups are represented at the bottom.
   Bottom: The boxplot of the 4-gene mRNA sigature score for 28 evaluated patients.

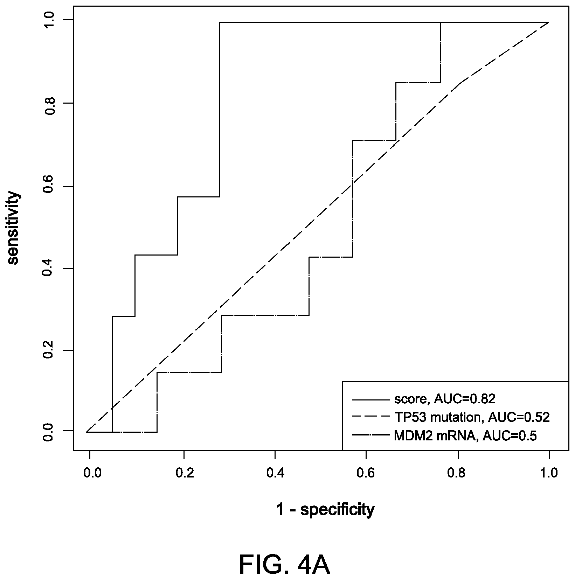

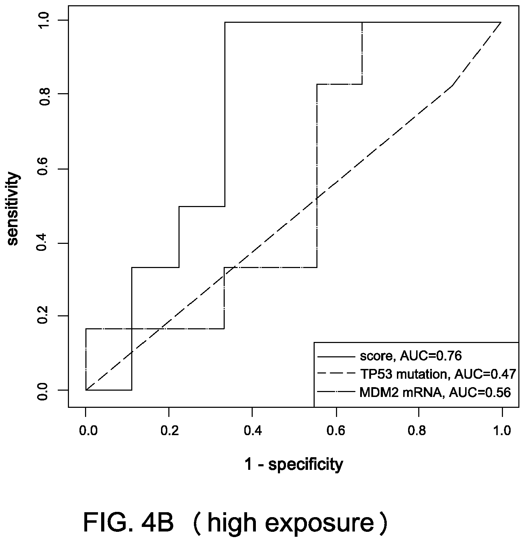

Figure 4A and 4B: The area under the curve (AUC) of receiver operating characteristic (ROC) curves of the three biomarks/biosignatures in AML trial. The four gene mRNA signature score showed AUC 0.82, MDM2 mRNA single biomarker showed AUC 0.5; P53 mutation status biomarker showed AUC 0.52.

### DETAILED DESCRIPTION OF THE INVENTION

In the context of the invention, an MDM2-inhibitor is any MDM2-inhibitor, preferably an MDM2-inhibitor specifically disclosed in U.S. Patent 8,354,444 B2, or in WO 2007/063013, WO 2010/031713, WO 2011/098398 and WO 2013/135648.

More specifically, an MDM2-inhibitor is a compound of formula I 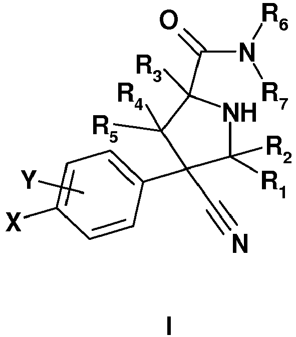 wherein
X is selected from the group consisting of H, F, Cl, Br, I, cyano, nitro, ethynyl, cyclopropyl, methyl, ethyl, isopropyl, vinyl and methoxy,
Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl, aryl, hetereoaryl, hetereocycle, COOR', OCOR', CONR'R", NR'COR", NR"SO₂R', SO₂NR'R" and NR'R" wherein
R' and R" are independently selected from H, lower alkyl, substituted lower alkyl, lower cycloalkyl, substituted lower cycloalkyl, lower alkenyl, substituted lower alkenyl, lower cycloalkenyl, substituted lower cycloalkenyl, aryl, substituted aryl, hetereoaryl, substituted hetereoaryl, hetereocycle, or substituted hetereocycle.
and in the case of R' and R" may independently link to form a cyclic structure selected from substituted or unsubstituted cycloalkyl, substituted or unsubstituted cycloalkenyl, substituted or unsubstituted heteroaryl or substituted or unsubstituted heterocycle,
one of R₁ and R₂ is selected from the group consisting of lower alkyl,
substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen or lower alkyl, R₃ is H or lower alkyl,
one of R₄ and R₅ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen,
R₆ and R₇ are selected from the group consisting of (CH₂)ₙ-R' (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ (CH₂)ₙ-COR',(CH₂)ₚ-(CH₂CH₂O)ₘ-(CH₂)ₙ-SONR'R",(CH₂)ₚ-(CH₂CH₂O)ₘ-(CH₂)ₙ-SO₂NR'R", -COR', -SOR' and SO₂R' wherein R' and R" are as above,
m, n and p are independently 0 to 6, and
the pharmaceutically acceptable salts and esters thereof.

Preferred compounds of formula I may have a stereochemical structure shown as formula II:

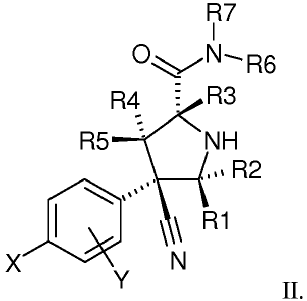

Another MDM2-inhibitor in the context of the present invention is a compound of formula IIa 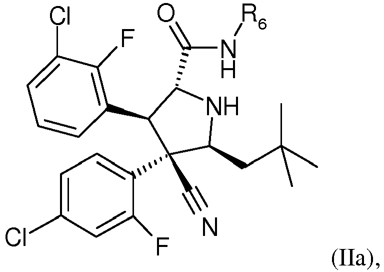 wherein
R₆ is -(CH₂)ₙ-R', and
R' is Cyclohexyl, or
   a 5 to 10 membered, mono- or bicyclic aromatic hydrocarbon wherein 1 or 2 carbon atoms may be replaced by N, S or O, and wherein any of the aforementioned cyclohexyl or aromatic hydrocarbon can be substituted once or twice with a group independently selected from lower alkyl, lower-alkenyl, lower-alkynyl, dioxo-lower-alkylene (forming e.g. a benzodioxyl group), halogen, hydroxy, CN, CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower-alkyl)₂-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)₂; and
n is 0 or 1.

The compounds of formula IIa are preferred, wherein
R₆ is -(CH₂)ₙ-R', and
R' is phenyl, pyridinyl, pyrazinyl or pyrimidinyl which can be each unsubstituted or once or twice substituted with a substituent independently selected from halogen, C1-6 alkoxy, C1-6 alkyl, hydroxycarbonyl, carboxy, carboxy CI-6 alkoxy, oxo and CN; and n is 0.

Another MDM2-inhibitor in the context of the present invention is a compound of formula III 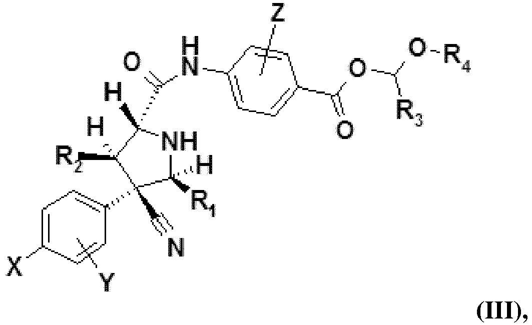 wherein
X is selected from the group consisting of H, F, Cl, Br, I, cyano, nitro, ethynyl, cyclopropyl, methyl, ethyl, isopropyl, vinyl and methoxy,
Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl,
Z is lower alkoxy,
R₁ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl,
R₂ is a substituted phenyl selected from: 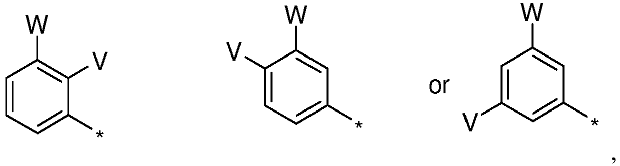
W is F, Cl or Br,
V is H or F,
R₃ is selected from the group consisting of hydrogen, lower alkyl or substituted lower alkyl,
R₄ is selected from the group consisting of: 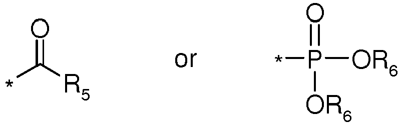
R₅ is selected from the group consisting of lower alkyl, substituted lower alkyl, heterocycle, substituted heterocycle, cycloalkyl, substituted cycloalkyl, natural and unnatural amino acids, -(OCH₂CH₂)ₙ-OH, -(OCH₂CH₂)ₙ-OCH₃, -(NCH₂CH₂)ₙ-OH, -(NCH₂CH₂)ₙ-OCH₃ and -(OCH₂CH₂)ₙ-OP(O)(OR₆)₂, wherein
n is from 3 to 60, preferably from 3 to 45,
R₆ is hydrogen or benzyl; or
a pharmaceutically acceptable salt or ester thereof.

Compounds of formula III are preferred, wherein
X is selected from H, F or Cl,
Y is selected from H, F or Cl,
R₁ is lower alkyl or substituted lower alkyl,
R₃ is hydrogen or lower alkyl,
R₅ is selected from the group consisting of lower alkyl, substituted lower alkyl, natural and unnatural amino acids, -(OCH₂CH₂)ₙ-OH, -(OCH₂CH₂)ₙ-OCH₃, -(NCH₂CH₂)ₙ-OH, - (NCH₂CH₂)ₙ-OCH₃, -(OCH₂CH₂)ₙ-OP(O)(OR₆)₂, wherein
n is from 3 to 60, preferably from 3 to 45, and
R₆ is hydrogen; or
a pharmaceutically acceptable salt thereof.

Compounds of formula III are prefedrred, wherein
X is selected from H, F or Cl;
Y is selected from H, F or Cl;
Z is CI-6 alkoxy;
R₁ is CI-6 alkyl;
R₂ is 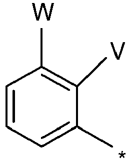 , wherein
W is F, Cl or Br;
V is H or F;
R₃ is hydrogen or CI-6 alkyl;
R₄ is -C(O)-R₅; wherein
R₅ is selected from the group consisting of -(OCH₂CH₂)ₙ-OH; -(OCH₂CH₂)ₙ-OCH₃; and -(OCH₂CH₂)ₙ-OP(O)(OR₆)₂, wherein n is from 3 to 60, and R₆ is hydrogen; or
a pharmaceutically acceptable salt thereof.
More specifically n is from 3 to 55, more preferably n is from 3 to 45. Within this embodiment, compounds wherein R₅ is -(OCH₂CH₂)ₙ-OCH₃ and n is from 40 to 60 are especially preferred.

In the specification where indicated the various groups may be substituted by 1-5 or, preferably, 1-3 substituents independently selected from the group consisting of lower alkyl, lower-alkenyl, lower-alkynyl, dioxo-lower-alkylene (forming e.g. a benzodioxyl group), halogen, hydroxy, CN, CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower-alkyl)₂-lower-alkoxy, lower-alkyl-1-oxiranyl-lower-alkoxy-lower-alkyl, 2-oxopyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)₂. Preferred substituents for the cycloalkyl, cycloalkenyl, aryl, heteroaryl and heterocycle rings are halogen, lower alkoxy, lower alkyl, hydroxycarbonyl, carboxy, carboxy lower alkoxy, oxo and CN. Preferred substituents for alkyl are alkoxy and N(lower alkyl)₂.

The term "alkyl" refers to straight- or branched-chain saturated hydrocarbon groups having from 1 to about 20 carbon atoms, including groups having from 1 to about 7 carbon atoms. In certain embodiments, alkyl substituents may be lower alkyl substituents. The term "lower alkyl" refers to alkyl groups having from 1 to 6 carbon atoms, and in certain embodiments from 1 to 4 carbon atoms. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 6, preferably 2 to 4 carbon atoms. Examples of such "alkenyl group" are vinyl ethenyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine, bromine, or iodine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl. Where the aryl group is bicyclic a preferred group is 1,3-dioxo-2,3-dihydro-1H-isoindol-5-yl group.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole substituted or unsubstituted triazolyl and substituted or unsubstituted tetrazolyl.

In the case of aryl or heteroaryl which are bicyclic it should be understood that one ring may be aryl while the other is heteroaryl and both being substituted or unsubstituted.

"Heterocycle" or "heterocyclic ring"means a substituted or unsubstituted 5 to 8 membered, mono- or bicyclic, non-aromatic hydrocarbon, wherein 1 to 3 carbon atoms are replaced by a hetero atom selected from nitrogen, oxygen or sulfur atom. Examples include pyrrolidin-2-yl; pyrrolidin-3-yl; piperidinyl; morpholin-4-yl and the like which in turn can be substituted. "Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, e.g. ethoxy ethoxy, methoxy ethoxy, methoxy ethoxy ethoxy and the like and substituted alkoxy side chains, e.g., dimethylamino ethoxy, diethylamino ethoxy, dimethoxy-phosphoryl methoxy and the like.

The term "mPEG" as used herein means methoxy polyethylene glycol, which is commercially available (e.g. Sigma-Aldrich or ID Biochem (Korea)). The molecular weight distribution of mPEG may vary according to the manufacturer and/or batch. In one embodiment of the present invention, mPEG has an average molecular weight (MW) of about 1500 Da to about 3000 Da. In another embodiment of the present invention mPEG has an average MW of about 2000 Da and about 2200 Da. Average MW is determined by MALDI-TOF mass spectrometry.

"Pharmaceutically acceptable derivative," such as pharmaceutically acceptable salts & esters, carrier, excipient, means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See*, *e*.*g*., Ansel et al., Pharmaceutical Dosage Forms and Drug Delivery Systems (6th Ed. 1995) at pp. 196 and 1456- 1457.

In one embodiment, an MDM2-inhibitor for use in the method according to present invention is 4-1[(2R,3S,4R,5S)-4-(4-Chloro-2-fluoro-phenyl)-3-(3-chloro-2-fluorophenyl)-4-cyano-5-(2,2-dimethyl-propyl)-pyrrolidine-2-carbonyl]-amino}-3-methoxy-benzoic acid of the formula 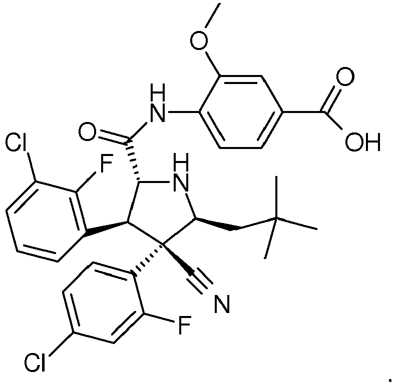 This compound and methods for making it are for example disclosed in WO 2011/098398.

Another specific compound in the context of the present invention is 2-((1-(4-((2R,3S,4R,5S)-3-(3-Chloro-2-fluorophenyl)-4-(4-chloro-2-fluorophenyl)-4-cyano-5-neopentylpyrrolidine-2-carboxamido)-3-methoxybenzoyloxy)ethoxy)carbonylamino)acetic acid.

Another specific compound in the context of the present invention is 2-(((4-((2R,3S,4R,5S)-3-(3-Chloro-2-fluorophenyl)-4-(4-chloro-2-fluorophenyl)-4-cyano-5-neopentylpyrrolidine-2-carboxamido)-3-methoxybenzoyloxy)methoxy)-carbonylamino)acetic acid.

A further compound in the context of the present invention is 3-Oxo-2,4,7,10,13,16,19-heptaoxaicosyl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate.

A specific compound in accordance with the present invention is 4-1[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, -2000).

A specific compound in accordance with the present invention is 4-1[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, -2200)

A specific compound in accordance with the present invention is 4-1[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-methyl ester (mPEG, average MW, -2000).

A specific compound in the context of the present invention is 3-Oxo-2,4,7,10,13-pentaoxatetradecyl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate;

A specific compound in the context of the present invention is 27-Oxo-2,5,8,11,14,17,20,23,26,28-decaoxatriacontan-29-yl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate.

A specific compound in the context of the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-[(2R,3R,4R,5S)-2-((R)-1,2-dihydroxy-ethyl)-4,5-dihydroxy-tetrahydro-furan-3-yloxycarbonyloxy]-ethyl ester.

Another specific compound in the context of the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-(2-{2-[2-(2-dibenzyloxyphosphoryloxy-ethoxy)-ethoxy]-ethoxy}-ethoxycarbonyloxy)-ethyl ester.

Also, a specific compound in the context of the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino }-3-methoxy-benzoic acid 1-(2-{2-[2-(2-phosphonooxy-ethoxy)-ethoxy]-ethoxy}-ethoxycarbonyloxy)-ethyl ester.

Also, a specific compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl] -amino} -3-methoxy-benzoic acid 1-mPEG-carbonyloxy-ethyl ester (mPEG, average MW, -2000) 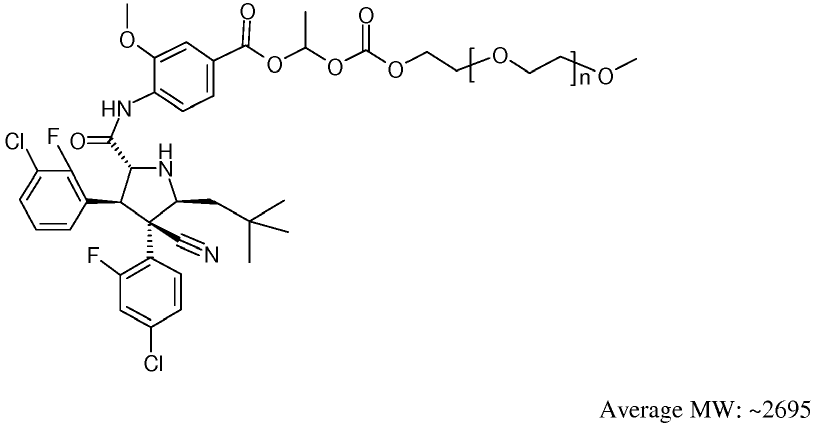 This compound and methods for making it are for example disclosed in WO 2013/135648

Also, a specific compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl] -amino} -3-methoxy-benzoic acid 1-mPEG-carbonyloxy-ethyl ester (mPEG, average MW, -2200) 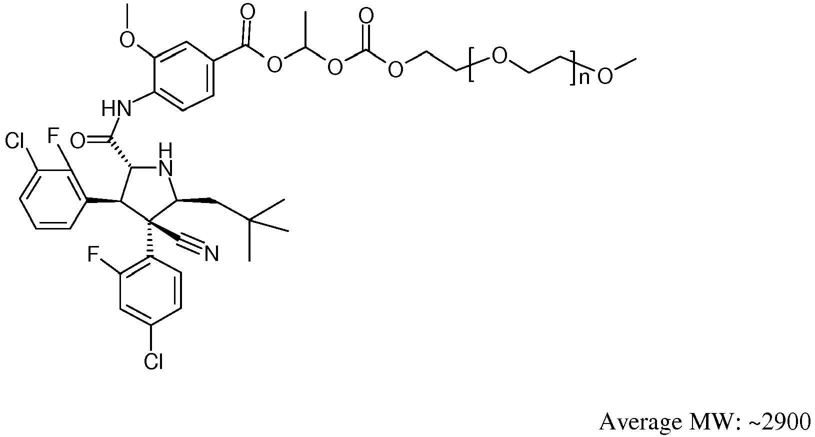 This compound and methods for making it are for example disclosed in WO 2013/135648

Also, a specific compound in accordance with the present invention is the compound of formula (A), also designated as Compound A (RG7112) 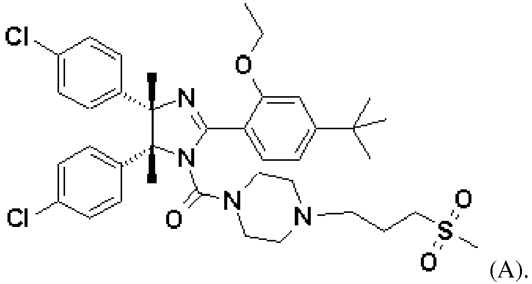 This compound and methods of making it are for example disclosed in WO 2007/063013.

### Establishment of MDM2 antagonist therapy predictive signature in cancer cell line collections.

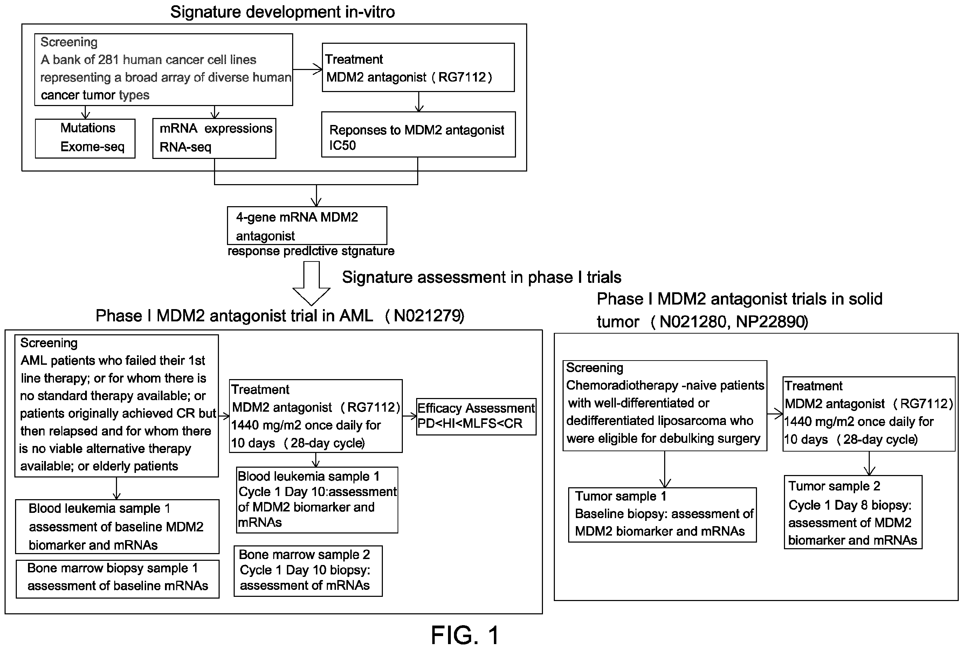

To generate an MDM2 antagonist therapy predictive signature, the response of MDM2 antagonist therapy *in vitro* with gene expression profiling (Figure 1) is combined. A bank of 281 human cancer cell lines, collectively termed the Cell Lines for Oncology (CELLO), representing a broad array of diverse human cancer tumor types are subject to evaluation using several high-throughput genomic technology platforms. The mutational status of nucleic acids from each cell line is determined by exome sequencing. Messenger RNA (mRNA) expression levels at baseline, prior to MDM2 antagonist therapy, are obtained via RNA sequencing. MDM2 antagonist therapy COMPOUND A, sometimes referred to herein as RG7112, responses are obtained by assays known to those skilled in the art such as RNA sequencing and microarray measurement using Gene Chip Human Genome U133 Plus 2.0 Array. The MDM2 therapy responses are obtained by high throughout screening assays. Depending on their responses to Compound A these cell lines are classified as either sensitive, defined as IC50 <=1, or resistant, defined as IC50 >=10.

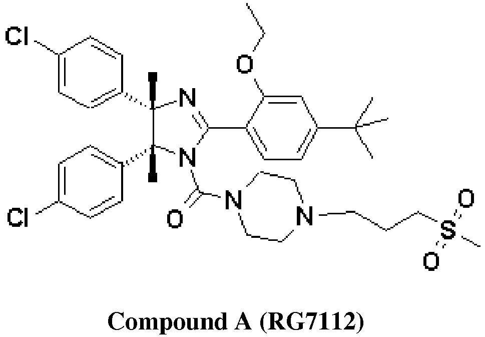

**Table 1: CELLO Cell Lines**

| Cell lines | Tissue | Origin | IC50 |
|---|---|---|---|
| 22Rvl | Prostate | Carcinoma | 0.97 |
| A-172 | Brain | Glioblastoma | 0.58 |
| A-375 | Skin | Melanoma, malignant | 0.32 |
| A549 | Lung | Carcinoma, squamous cell | 0.86 |
| ACHN | Kidney | Adenocarcinoma, renal cell | 0.29 |
| AGS | Stomach | Adenocarcinoma, gastric | 0.13 |
| Caki-1 | Kidney | Carcinoma, clear cell | 0.37 |
| D341 Med | Brain, cerebellum | Medulloblastoma | 0 |
| HCT116 | Intestine, large; colon | Carcinoma, colorectal | 0.5 |
| HepG2 | Liver | Carcinoma, hepatocellular | 0.18 |
| Hs 38.T | Ovary | Teratoma | 0.73 |
| HT-1080 | Connective tissue | Fibrosarcoma | 0.058 |
| HT-1197 | Urinary, bladder | Carcinoma, urinary bladder, papillary (PAP) | 0.19 |
| IM-9 | Lymphocyte B, peripheral blood | Myeloma, multiple | 0.14 |
| IMR-32 | Brain | Neuroblastoma (Glioma, neuroblastoma) | 0.43 |
| KMS-12-BM | Bone marrow | Myeloma, multiple | 0 |
| KMS-21BM | Bone marrow | Myeloma, multiple | 0 |
| KMS-26 | Lymphocyte | Myeloma | 0.45 |
| LCLC-103H | Lung | Carcinoma, large cell, non-small cell lung cancer | 0.7 |
| LN-18 | Brain, temporal lobe | Glioblastoma | 0.67 |
| LNCaP clone FGC | NA | NA | 0.17 |
| LS 174T | Intestine, large; colon | Adenocarcinoma, colorectal | 0.59 |
| LS513 | Intestine, large; caecum | Carcinoma, colorectal | 0.49 |
| MC/CAR | Lymphocyte B, peripheral blood | Myeloma, plasmacytoma | 0.69 |
| MCF7 | Breast, mammary gland | Adenocarcinoma, mammary gland, breast | 0.75 |
| MKN-45 | Stomach | Adenocarcinoma | 0.18 |
| MKN-74 | Muscle, smooth, stomach | Carcinoma, gastric | 0.88 |
| MOLM-13 | Blood | Leukemia, acute myeloid | 0.19 |
| MV-4-11 | Blood | Leukemia, acute monocytic | 0 |
| NCI-H2122 | Lung | Adenocarcinoma | 0.16 |
| NCI-H226 | Lung | Carcinoma, squamous cell | 0.13 |
| NCI-H28 | null | Mesothelioma | 0.46 |
| NCI-H460 | Lung | Carcinoma | 0.88 |
| NCI-H929 | Lymphocyte B, bone marrow | Myeloma, plasmacytoma | 0.3 |
| NKM-1 | Lymphocyte | Leukemia, acute myeloid | 0.32 |
| NUGC-4 | Stomach | Adenocarcinoma | 0.38 |
| PA-1 | Ovary | Carcinoma, ovary | 0.62 |
| PC-9 | Lung | Adenocarcinoma | 0.69 |
| RKO | Intestine, large; colon | Carcinoma, colorectal | 0 |
| RPMI-2650 | Nasal septum | Carcinoma, squamous cell | 0.77 |
| SH-SY5Y | Brain | Neuroblastoma (Glioma, neuroblastoma) | 0.75 |
| SK-HEP-1 | Liver | Hepatoma, Hepatocellular carcinoma, HCC | 0.22 |
| SK-MEL-1 | Skin | Melanoma, malignant | 0.75 |
| SK-N-SH | Brain | Neuroblastoma (Glioma, neuroblastoma) | 0.21 |
| SNU-1 | Stomach | Carcinoma, gastric | 0.37 |
| SR | Lymphocyte | Lymphoma, large cell | 0.043 |
| SW780 | Urinary, bladder | Carcinoma, urinary bladder, transitional cell carcinoma (TCC) | 0.97 |
| U-87 MG | Brain | Glioblastoma | 0.18 |
| WERI-Rb-1 | Eye, retina | Retinoblastoma | 0.67 |
| Y79 | Eye, retina | Retinoblastoma | 0.61 |
| ZR-75-1 | Breast, mammary gland | Carcinoma, ductal | 0.076 |
| 5637 | Urinary, bladder | Carcinoma, urinary bladder, papillary (PAP) | 8.5 |
| 143B | Bone | Osteosarcoma | 15 |
| 647-V | Urinary tract, urothelium | Carcinoma, urinary bladder, transitional cell carcinoma (TCC) | 17 |
| 786-O | Kidney | Adenocarcinoma, renal cell | 9.5 |
| A-204 | Muscle | Rhabdomyosarcoma | 8.4 |
| A2058 | Skin | Melanom | 17 |
| A-431 | Skin, epidermis | Carcinoma, epidermoid | 9.5 |
| ABC-1 | Lung | Adenocarcinoma | 9.9 |
| ARH-77 | Lymphocyte B, peripheral blood | Leukemia, plasma cell | 17 |
| BT-20 | Breast, mammary gland | Adenocarcinoma, mammary gland, breast | 3.6 |
| C32 | Skin | Melanoma | 3 |
| Calu-1 | Lung | Carcinoma, lung epidermoid OR Carcinoma, epidermoid pulmonary | 14 |
| Calu-3 | Lung | Adenocarcinoma | 11 |
| Calu-6 | Lung | Carcinoma, anaplastic | 17 |
| Capan-2 | Pancreas | Adenocarcinoma | 9.1 |
| CCRF-CEM | Lymphocyte T, peripheral blood | Leukemia, acute lymphoid | 9.7 |
| CEM/C2 | Lymphocyte T, peripheral blood | Leukemia, acute lymphoid | 9.3 |
| CFPAC-1 | Pancreas | Adenocarcinoma, ductal, cystic fibrosis | 5.7 |
| CMK-11-5 | Lymphocyte | Leukemia, acute megakaryoblastic | 9.6 |
| COLO 201 | Intestine, large; colon | Adenocarcinoma, colorectal | 10 |
| COLO 205 | Intestine, large; colon | Adenocarcinoma, colorectal | 9.1 |
| COLO 320DM | Intestine, large; colon | Adenocarcinoma, colorectal | 12 |
| COLO-824 | Breast | Carcinoma | 15 |
| D-283MED | NA | NA | 1.2 |
| Daoy | Brain, cerebellum | Medulloblastoma, desmoplastic cerebellar | 9.7 |
| Detroit 562 | Pharynx | Carcinoma | 6.2 |
| DLD-1 | Intestine, large; colon | Adenocarcinoma | 5.1 |
| DMS 114 | Lung | Carcinoma, classic small cell lung cancer | 10 |
| DU145 | Prostate | Carcinoma, prostate | 1 |
| DU-4475 | Breast, mammary gland | Carcinoma | 2.1 |
| EGI-1 | Bile duct | Carcinoma, bile duct OR Cholangiocarcinoma | 9 |
| ES2 | Ovary | Carcinoma, clear cell | 3.3 |
| HARA-B | Lung | Carcinoma, squamous cell | 10 |
| HCC1143 | Breast, mammary gland | Carcinoma, primary ductal | 12 |
| HCC1187 | Breast, mammary gland | Carcinoma, primary ductal | 12 |
| HCC1500 | Breast, mammary gland | Carcinoma, primary ductal | 1 |
| HCC1569 | Breast, mammary gland | Carcinoma, primary metaplastic | 15 |
| HCC1599 | Breast, mammary gland | Carcinoma, primary ductal | 12 |
| HCC1806 | Breast, mammary gland | Carcinoma, primary acantholytic squamous cell | 11 |
| HCC1937 | Breast | Carcinoma, ductal | 15 |
| HCC1954 | Breast, mammary gland | Carcinoma, ductal | 12 |
| HCC366 | Lung | Adenocarcinoma, squamous cell, non-small cell lung cancer | 8.7 |
| HCC38 | Breast, mammary gland | Carcinoma, primary ductal | 12 |
| HCC827 | Lung | Adenocarcinoma | 8.9 |
| HCT-15 | Intestine, large; colon | Adenocarcinoma, colorectal | 7.6 |
| HCT-8 | Intestine, large; colon | Adenocarcinoma, colorectal, ileocecal | 16 |
| HEC-151 | Uterus | Adenocarcinoma, malignant, endometroid carcinoma | 2.2 |
| HEC-1-A | Uterus, endometrium | Adenocarcinoma | 16 |
| HEL | Lymphocyte, peripheral blood | Erythroleukemia OR Leukemia, erythroid | 17 |
| Hep3B | Liver | Carcinoma, hepatocellular | 6.7 |
| HL-60 | Blood | Leukemia, acute promyeloid | 7 |
| HMCB | Skin | Melanoma | 15 |
| HPAC | Pancreas | Adenocarcinoma | 4.6 |
| HPAF-II | Pancreas | Adenocarcinoma | 10 |
| Hs 578T | Breast, mammary gland | Carcinoma | 8.8 |
| Hs 766T | Pancreas | Carcinoma | 17 |
| HT-1376 | Urinary, bladder | Carcinoma | 16 |
| HT-29 | Intestine, large; colon | Adenocarcinoma | 7.4 |
| HuH-28 | Gall bladder | Carcinoma, bile duct OR Cholangiocarcinoma | 15 |
| Huh-7 | Liver | Hepatoma, Hepatocellular carcinoma, HCC | 10 |
| HUP-T4 | Pancreas | Carcinoma, pancreatic | 17 |
| JIMT-1 | Breast | Carcinoma | 11 |
| K-562 | Bone marrow | Leukemia, chronic myeloid | 16 |
| KATO-III | Stomach | Carcinoma, gastric | 9.7 |
| KELLY | Brain | Neuroblastoma (Glioma, neuroblastoma) | 15 |
| KG-1 | Bone marrow | Leukemia | 15 |
| KG-la | Bone marrow | Leukemia, acute myeloid | 9 |
| KHM-1B | Lymphocyte | Myeloma, multiple | 6.9 |
| KMS-11 | Lymphocyte | Myeloma, multiple | 12 |
| KMS-12-PE | Plasma | Myeloma, multiple | 5.3 |
| KMS-20 | Lymphocyte | Myeloma | 13 |
| KMS-28BM | Lymphocyte | Myeloma | 9 |
| KMS-34 | Lymphocyte | Myeloma | 9.8 |
| KYSE-150 | Esophagus; oesophagus | Carcinoma, squamous cell | 3 |
| KYSE-520 | Esophagus; oesophagus | Carcinoma, squamous cell | 10 |
| LoVo | Intestine, large; colon | Adenocarcinoma, colorectal | 1.3 |
| LP-1 | Lymphocyte, peripheral blood | Myeloma, multiple | 4.4 |
| LS1034 | Intestine, large; caecum | Carcinoma, colorectal | 5.7 |
| LS411N | Intestine, large; caecum | Carcinoma, colorectal | 16 |
| MCF10DCIS.com | Breast | Carcinoma | 1.4 |
| MDA-MB-231 | Breast, mammary gland | Adenocarcinoma | 17 |
| MDA-MB-435S | Skin | Melanoma | 12 |
| MDA-MB-468 | Breast, mammary gland | Adenocarcinoma, mammary gland, breast | 10 |
| ME-180 | Cervix | Carcinoma, epidermoid | 9.3 |
| MEG-01 | null | Leukemia, chronic myeloid | 17 |
| MFM-223 | Breast, mammary gland | Carcinoma, ductal | 17 |
| MG-63 | Bone | Osteosarcoma | 16 |
| MKN-1 | Muscle, smooth, stomach | Carcinoma, squamous cell | 15 |
| MKN-28 | Stomach | Carcinoma, gastric | 8.1 |
| NB-4 | Myeloblast | Leukemia, acute promyeloid | 4.7 |
| NCCIT | Germ cell, gametocyte | Teratocarcinoma | 11 |
| NCI-H1048 | Lung | Carcinoma, small cell lung cancer | 9.4 |
| NCI-H1299 | Lung | Carcinoma, non-small cell lung cancer | 15 |
| NCI-H1395 | Lung | Adenocarcinoma | 1.3 |
| NCI-H1437 | Lung | Adenocarcinoma, non-small cell lung cancer | 10 |
| NCI-H146 | Lung | Carcinoma, small cell lung cancer | 8.3 |
| NCI-H1568 | NA | NA | 9.6 |
| NCI-H1650 | Lung | Adenocarcinoma | 10 |
| NCI-H1666 | Lung | Adenocarcinoma | 13 |
| NCI-H1703 | Lung | Adenocarcinoma, non-small cell lung cancer | 9.1 |
| NCI-H1755 | Lung | Adenocarcinoma, non-small cell lung cancer | 7.8 |
| NCI-H1781 | Lung | Carcinoma, bronchioalveolar, non-small cell lung cancer | 8.8 |
| NCI-H1792 | Lung | Adenocarcinoma | 7 |
| NCI-H1793 | Lung | Adenocarcinoma, non-small cell lung cancer | 10 |
| NCI-H1838 | Lung | Adenocarcinoma, non-small cell lung cancer | 9.8 |
| NCI-H187 | Lung | Carcinoma, classic small cell lung cancer | 15 |
| NCI-H1944 | Lung | Adenocarcinoma, non-small cell lung cancer | 7 |
| NCI-H1975 | Lung | Adenocarcinoma | 16 |
| NCI-H1993 | Lung | Adenocarcinoma, non-small cell lung cancer | 7.7 |
| NCI-H2009 | Lung | Adenocarcinoma | 9.9 |
| NCI-H2023 | Lung | Adenocarcinoma, non-small cell lung cancer | 5.8 |
| NCI-H2029 | Lung | Carcinoma, small cell lung cancer | 5.8 |
| NCI-H2030 | Lung | Adenocarcinoma, non-small cell lung cancer | 9.2 |
| NCI-H2081 | Lung | Carcinoma, small cell lung cancer | 17 |
| NCI-H209 | Lung | Carcinoma, small cell lung cancer | 11 |
| NCI-H2170 | Lung | Carcinoma, squamous cell | 12 |
| NCI-H2171 | Lung | Carcinoma, small cell lung cancer | 11 |
| NCI-H2227 | Lung | Carcinoma, small cell lung cancer | 16 |
| NCI-H2228 | Lung | Adenocarcinoma, non-small cell lung cancer | 9.8 |
| NCI-H23 | Lung | Adenocarcinoma, non-small cell lung cancer | 10 |
| NCI-H2347 | Lung | Adenocarcinoma, non-small cell lung cancer | 9 |
| NCI-H250 | Lung | Carcinoma, classic small cell lung cancer | 12 |
| NCI-H292 | Lung | Carcinoma, mucoepidermoid pulmonary | 1.6 |
| NCI-H345 | Lung | Carcinoma, small cell lung cancer | 17 |
| NCI-H358 | Lung, bronchi | Carcinoma, alveola cell | 8.1 |
| NCI-H441 | Lung | Adenocarcinoma | 10 |
| NCI-H508 | Intestine, large; caecum | Adenocarcinoma, colorectal | 1.2 |
| NCI-H520 | Lung | Carcinoma, squamous cell | 17 |
| NCI-H526 | Lung | Carcinoma, small cell lung cancer | 16 |
| NCI-H661 | Lung | Carcinoma, large cell, neuroendocrine, non-small lung cancer | 11 |
| NCI-H69 | Lung | Carcinoma, small cell lung cancer | 15 |
| NCI-H716 | Intestine, large; caecum | Adenocarcinoma, colorectal | 13 |
| NCI-H748 | Lung | Carcinoma, small cell lung cancer | 16 |
| NCI-H82 | Lung | Carcinoma, small cell lung cancer | 4.9 |
| NCI-H838 | Lung | Adenocarcinoma, non-small cell lung cancer | 14 |
| NCI-N87 | Stomach | Carcinoma, gastric | 12 |
| NOMO-1 | Bone marrow | Leukemia, acute myeloid | 6.3 |
| NUGC-3 | Stomach | Adenocarcinoma | 9.8 |
| OPM-2 | Lymphocyte B | Myeloma, multiple | 13 |
| NIH:OVCAR-3 | Ovary | Adenocarcinoma | 17 |
| PC-1 | null | null | 1.3 |
| PC-10 | Lung | Carcinoma, squamous cell | 14 |
| PC-13 | Lung | Adenocarcinoma | 14 |
| PC-3 | Prostate | Adenocarcinoma | 9 |
| PC-6 | Lung | Carcinoma, small cell lung cancer | 8.2 |
| PLC/PRF/5 | Liver | Hepatoma | 7 |
| QG-56 | Lung | Carcinoma, non-small cell lung cancer | 10 |
| Raji | Lymphocyte B | Lymphoma, Burkitt | 3 |
| Ramos | Lymphocyte B | Lymphoma, Burkitt | 8.8 |
| RL95-2 | Uterus, endometrium | Carcinoma | 9.3 |
| RPMI-8226 | Lymphocyte B, peripheral blood | Myeloma, plasmacytoma | 6.6 |
| RT4 | Urinary, bladder | Papilloma, transitional cell | 2.4 |
| SCaBER | Urinary, bladder | Carcinoma, squamous cell | 8.5 |
| SCC-25 | Tongue | Carcinoma, squamous cell | 9.4 |
| SCH | Stomach | Choriocarcinoma | 17 |
| SJCRH30 | Muscle | Rhabdomyosarcoma | 16 |
| SK-LU-1 | Lung | Adenocarcinoma | 11 |
| SK-MES-1 | Lung | Carcinoma, squamous cell | 6 |
| SK-N-AS | Brain | Neuroblastoma (Glioma, neuroblastoma) | 13 |
| SK-N-DZ | Brain | Neuroblastoma (Glioma, neuroblastoma) | 5.7 |
| SK-N-F1 | Brain | Neuroblastoma (Glioma, neuroblastoma) | 17 |
| SK-OV-3 | Ovary | Adenocarcinoma | 17 |
| SNU-16 | Stomach | Carcinoma, gastric | 10 |
| SNU-5 | Stomach | Carcinoma, gastric | 16 |
| SU.86.86 | Pancreas | Carcinoma, ductal | 6.7 |
| SUM-44PE | Breast | Carcinoma | 3 |
| SUM52PE | Breast | Carcinoma | 8.7 |
| SW1116 | Intestine, large; colon | Adenocarcinoma, colorectal | 12 |
| SW1417 | Intestine, large; colon | Adenocarcinoma, colorectal | 9.9 |
| SW1463 | Intestine, large; rectum | Adenocarcinoma, colorectal | 1 |
| SW403 | Intestine, large; colon | Adenocarcinoma, colorectal | 6 |
| SW480 | Intestine, large; colon | Adenocarcinoma, colorectal | 12 |
| SW579 | Thyroid gland | Carcinoma, squamous cell | 16 |
| SW620 | Intestine, large; colon | Adenocarcinoma, colorectal | 9.8 |
| SW626 | Ovary | Adenocarcinoma | 9.5 |
| SW837 | Intestine, large; rectum | Adenocarcinoma | 12 |
| T-47D | Breast, mammary gland | Carcinoma, ductal | 9.9 |
| T84 | Intestine, large; colon | Carcinoma, colorectal | 5.3 |
| T98G | Brain | Glioblastoma, multiforme | 8.3 |
| TCCSUP | Urinary, bladder | Carcinoma, urinary bladder, transitional cell carcinoma (TCC) | 16 |
| TF-la | Bone marrow | Erythroleukemia OR Leukemia, erythroid | 10 |
| THP-1 | Blood | Leukemia, acute monocytic | 9.7 |
| TT | Thyroid gland | Carcinoma | 2 |
| U-698-M | Tonsil | Lymphoma, lymphoblastic, non-Hodgkin's | 9.8 |
| U-937 | Pleura | Lymphoma, histiocytic | 5.2 |
| UM-UC-3 | Urinary, bladder | Carcinoma, urinary bladder, transitional cell carcinoma (TCC) | 15 |
| VCaP | Prostate | Carcinoma, prostate | 13 |
| WiDr | Intestine, large; colon | Adenocarcinoma, colorectal | 8.9 |
| 10C9 | Lymphocyte B | Lymphoma, Non-Hodgkin's | 19 |
| 8305C | Thyroid gland | Carcinoma, medullary thyroid | 24 |
| A-673 | Muscle | Rhabdomyosarcoma | 18 |
| AN3 CA | Uterus, endometrium | Adenocarcinoma | 18 |
| AsPC-1 | Pancreas | Adenocarcinoma | 19 |
| BFTC-905 | Urinary, bladder | Carcinoma, urinary bladder, transitional cell carcinoma (TCC) | 19 |
| BT-474 | Breast, mammary gland | Carcinoma, ductal | 19 |
| BT-483 | Breast, mammary gland | Carcinoma, ductal | 20 |
| BxPC-3 | Pancreas | Adenocarcinoma | 18 |
| C-33 A | Cervix | Carcinoma, cervix | 18 |
| CAMA-1 | Breast, mammary gland | Adenocarcinoma, mammary gland, breast | 22 |
| Capan-1 | Pancreas | Adenocarcinoma | 20 |
| COLO-704 | Ovary | Carcinoma, ovary | 20 |
| EFO-21 | Ovary | Carcinoma, ovary | 31 |
| F-36P | Myeloblast | Leukemia, acute myeloid | 23 |
| HARA | Lung | Carcinoma, squamous cell | 19 |
| HCC1395 | Breast, mammary gland | Carcinoma, primary ductal | 20 |
| HDLM-2 | Lymphocyte B | Lymphoma, Hodgkin's disease | 33 |
| HeLa S3 | Cervix | Carcinoma, cervix | 20 |
| J82 | Urinary, bladder | Carcinoma, urinary bladder, transitional cell carcinoma (TCC) | 20 |
| KARPAS-299 | Blood | Lymphoma | 19 |
| Kasumi -1 | Lymphocyte, peripheral blood | Leukemia, acute myeloid | 21 |
| KMM-1 | Lymphocyte | Myeloma | 19 |
| L-363 | Lymphocyte, peripheral blood | Leukemia, plasma cell | 32 |
| MDA-MB-134-VI | Breast, mammary gland | Carcinoma, ductal | 38 |
| MDA-MB-157 | Breast, mammary gland | Carcinoma, medula | 20 |
| MDA-MB-361 | Breast, mammary gland | Adenocarcinoma, mammary gland, breast | 21 |
| MDA-MB-453 | Breast, mammary gland | Carcinoma | 20 |
| MFE-280 | Uterus, endometrium | Adenocarcinoma, malignant, endometroid carcinoma | 23 |
| MIA PaCa-2 | Pancreas | Carcinoma | 19 |
| NCI-H1155 | Lung | Carcinoma, non-small cell lung cancer | 22 |
| NCI-H446 | Lung | Carcinoma, small cell lung cancer | 18 |
| NCI-H522 | Lung | Adenocarcinoma, non-small cell lung cancer | 22 |
| NCI-H596 | Lung | Carcinoma, adenosquamous | 18 |
| PANC-1 | Pancreas | Carcinoma, epithelioid | 22 |
| SBC-5 | Lung | Carcinoma, small cell lung cancer | 18 |
| SCC-15 | Tongue | Carcinoma, squamous cell | 20 |
| SK-BR-3 | Pleura | Adenocarcinoma, mammary gland, breast | 21 |
| SK-ES-1 | Bone | Sarcoma, Ewings | 25 |
| SKM-1 | Lymphocyte, peripheral blood | Leukemia, acute myeloid | 19 |
| SK-MEL-30 | Skin | Melanoma, malignant | 19 |
| SW948 | Intestine, large; colon | Adenocarcinoma, colorectal | 19 |
| T24 | Urinary, bladder | Carcinoma, urinary bladder, transitional cell carcinoma (TCC) | 21 |
| U-118 MG | Brain | Glioblastoma, astrocytoma | 19 |
| U-138 MG | Brain | Glioblastoma | 29 |
| U-2197 | Skin, hypodermis; subcutaneous | Histiocytoma, fibrous, malignant | 22 |
| U266B 1 | Lymphocyte B | Myeloma, plasmacytoma | 18 |
| YAPC | Pancreas | Carcinoma, pancreatic | 18 |

Among the 281 cell lines, 210 cell lines show mutations in TP53 after a careful annotation removing low quality calls and germline mutations. Cell lines harboring mutant TP53 are much less sensitive to MDM2 antagonist therapy (P < 2.2x10⁻¹⁶), consistent with previously published data. However, two challenges are observed in using TP53 mutation status as a potential predictive biomarker for COMPOUND A. Although not wanting to be limited by theory, first, several TP53 mutant cell lines show sensitive responses to COMPOUND A most likely because they harbor non-functional TP53 mutations, such as 22Rv1, DU145, KMS-12-BM, KMS-26, LCLC-103H, LN-18, MKN-45, NCI-H2122, NCI-H226, PC-9, SW1463.

Some other methods of assaying include but are not limited to a) immunohistochemistry (IHC) analysis, b) western blotting c) immunoprecipitation d) enzyme linked immunosorbant assay (ELISA) e) radioimmunoassy f) Fluorescence activated cell sorting (FACS) g) mass spectrometry, including matrix assisted laser desorpotion/ionisation (MALDI, e.g. MALDI-TOF) and electrospray ionisation mass-spectrometry (ESI--MS).

One approach for assessing potential response to a MDM2 antagonist is to assess whether or not the TP53 gene is mutated. However, this is complicated by the fact that a multitude of mutations can be found in TP53 in cancer. Not all of these mutations will interfere with activity of the p53 protein, further complicating the interpretation of TP53 mutational tests. In addition, there is a range of responses to MDM2 antagonists in wild type TP53 cell lines and patients. Therefore, the ability to predict responsiveness to an MDM2 antagonist from an easily interpretable diagnostic tool is an unmet need in clinical development of MDM2 antagonists. To this end, the development of a gene expression signature that reflects p53 pathway activity provides a means of selecting patients most likely to respond to MDM2 antagonist therapy.

Towards this end, a genome-wide association between baseline mRNA expression and MDM2 antagonist therapy response (IC50) is performed and identified a list of 13 genes with significant associations with P ranging from 2.38x10-47 to 9.56x10⁻²¹

Functional annotation indicates that the 13 significant genes from the genomewide association, with correlation coefficients ranging from -0.47 to -0.31 (with one positive correlation 0.28), are known regulators in the relevant MDM2-P53 interactions or downstream P53 pathways, including cell cycle arrest and apoptosis (see Table 2). Among them, MDM2 is the number 4 top gene with an over-expression of MDM2 correlating with *in vitro* sensitivity, consistent with previously published data. Therefore, disclosed herein is a method to predict responsiveness of a patient with cancer to a therapy, said method comprising detecting a level of at least one gene according to Table 2 and using that level as a biomarker for predicting said patients response to a compound, wherein the compound is an inhibitor of the MDM2-p53 interaction (MDM2-inhibitor). Also disclosed is a method for predicting said patients response to treatment with an MDM2-inhibitor, said method comprising detection of combined levels comprising 2, or 3, or 4, or 5, or 6, or 7, or 8, or 9, or 10, or 11, or 12, or detection of all 13 genes according to Table 2. The detection of said genes, or combination of genes, according to Table 2 can also be used to monitor response of said patient during treatment with said MDM2-inhibitor in order to decide whether said treatment shall continue ot not. Said method comprises the following steps:
a) taking a sample from the patient;
b) measuring a level of at least one gene according to Table 2, in the sample;
c) comparing said level from the patient to standard values, for example values obtained from a patient with the same cancer; and
d) administering a compound which acts as inhibitor of the MDM2-p53 interaction.
The sample in a) is obtained prior to start of treatment if the present method is applied as a predictive method for a patient's response to treatment, and during treatment if the present method is applied for treatment monitoring.
The term "detecting (or measuring) a level" in accordance with the present invention preferably means detecting (or measuring) mRNA expression levels. Also disclosed is at least one gene according to Table 2, or combinations thereof, for use as biomarker(s) for determining a cancer patient's response to treatment with an MDM2-inhibitor as defined herein. The term "cancer" is as defined herein, preferably AML (see page 4).

To further construct an mRNA signature, a multivariate logistic regression classifier is built, among the 13 genes, via upward model selection procedure. The mRNA signature is composed of up-regulations of three genes including MDM2, XPC (xeroderma pigmentosum, complementation group C), BBC3 (BCL2 binding component 3) and down-regulation of tumor suppressor gene CDKN2A cyclin-dependent kinase inhibitor 2a.

**TABLE 2: Significant gene expression predictors from genomedwide association**

| Ensg.id | Gene | Cor | Pvalue | Mean (Sen) | SD (Sen) | Mean (Res) | SD (Res) | FC | Annotation |
|---|---|---|---|---|---|---|---|---|---|
| ENSG00000087088 | BAX | -0.47 | 9.56E-23 | 5.12 | 0.63 | 4.48 | 0.59 | 1.56 | apoptosis |
| ENSG00000185088 | RPS27L | -0.45 | 2.83E-23 | 4.23 | 0.80 | 3.01 | 0.72 | 2.32 | apoptosis |
| ENSG00000131080 | EDA2R | -0.43 | 1.36E-24 | 0.28 | 3.59 | -4.98 | 2.36 | 38.23 | P53-related |
| ENSG00000154767 | XPC | -0.42 | 5.33E-24 | 3.56 | 0.44 | 2.72 | 0.62 | 1.79 | DNA repair |
| ENSG00000134574 | DDB2 | -0.41 | 4.72E-24 | 4.65 | 0.78 | 3.78 | 0.82 | 1.83 | DNA repair |
| ENSG00000161513 | FDXR | -0.41 | 3.59E-24 | 4.13 | 0.95 | 3.00 | 1.15 | 2.20 | P53-related |
| ENSG00000135679 | MDM2 | -0.39 | 1.12E-24 | 4.63 | 0.87 | 3.60 | 0.64 | 2.04 | |
| ENSG00000124762 | CDKN1A | -0.39 | 1.04E-24 | 5.51 | 1.59 | 3.54 | 2.01 | 3.92 | cell cycle arrest |
| ENSG00000170855 | TRIAP1 | -0.38 | 8.10E-25 | 5.53 | 0.49 | 5.04 | 0.49 | 1.40 | apoptosis |
| ENSG00000105327 | BBC3 | -0.34 | 9.26E-26 | 2.05 | 1.18 | 0.65 | 1.41 | 2.64 | apoptosis |
| ENSG00000113328 | CCNG1 | -0.31 | 1.47E-26 | 5.65 | 0.69 | 4.79 | 0.80 | 1.81 | cell cycle arrest |
| ENSG00000120889 | TNFRSF10B | -0.31 | 1.37E-26 | 4.69 | 1.02 | 3.50 | 1.41 | 2.28 | apoptosis |
| ENSG00000147889 | CDKN2A | 0.28 | 2.38E-47 | -1.71 | 3.79 | 1.35 | 4.26 | 0.12 | MDM2-related |

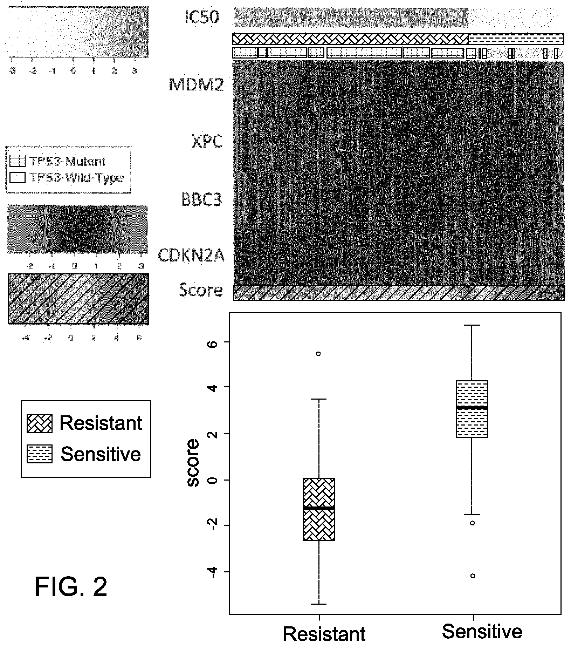

The final signature is capable of distinguishing MDM2 antagonist sensitive cell lines from MDM2 antagonist resistant cell lines with an area (AUC) under the receiver operating characteristic (ROC) curve of 0.93 (95% CI 0.92 to 0.95, Table 3), estimated from a 10-fold cross-validation. Therefore, the MDM2 antagonist sensitive cell lines demonstrate baseline up-regulation of MDM2, XPC, and BBC3 and down-regulation of CDKN2A; whereas the MDM2 antagonist resistant cell lines are characterized by down regulation of MDM2, XPC, and BBC3 and up-regulation of CDKN2A. (See Figure 2).

**TABLE 3: Prediction from various predictive biomarkers**

| | CELLO^{a} | NO21279^{b} |
|---|---|---|
| **Score** | | |
| AUC | 0.92 | 0.82 |
| Specificity^{c} | 0.67 | 0.71 |
| Sensitivity^{d} | 0.93 | 1.00 |

| **TP53** | | |
|---|---|---|
| AUC | 0.87 | 0.52 |
| Specificity^{e} | 0.95 | 0.19 |
| Sensitivity^{f} | 0.80 | 0.86 |

| **MDM2** | | |
|---|---|---|
| AUC | 0.83 | 0.50 |
| Specificity^{c} | 0.65 | 0.48 |
| Sensitivity^{d} | 0.85 | 0.43 |

| | | |
|---|---|---|
| a. Responders defined as IC50<1; Non-responders defined as IC50>10 in CELLO b. Responders defined as CR/MLFS; Non-responders defined as HI/PD in NO21279 c. Specificity: Proportion of non-responders that have scores or MDM2 expression lower than corresponding Youden Index d. Sensitivity: Proportion of responders that have scores or MDM2 expression higher than corresponding Youden Index e. Specificity: Proportion of non-responders that have TP53 mutations f. Sensitivity: Proportion of responders that have wild type TP53 | | |

In addition to the target gene MDM2, the other three genes in the signature are all biologically supported as regulators in the MDM2-p53 interactions or downstream p53 pathways. The XPC gene plays an important role involved in repairing damaged DNA, contributing to damage recognition, open complex formation, and repair protein complex formation. BBC3 mRNA levels are induced by exposure to DNA-damaging agents and by p53, which mediates DNA damage-induced apoptosis. The two gene products of the CDKN2A, p16 and p14ARF, are both linked to major tumor suppressor pathways; especially p14ARF, which inhibits MDM2 function by sequestering it in the nucleolus. To examine the molecular mechanisms underlying the mRNA signature, the mRNA signature score is correlated with mutation status of P53 and key regulatory genes involved in MDM2-P53 interactions and downstream P53 pathways.

As shown in Figure 2, cell lines with low signature score are more likely to be p53 mutant; whereas cell lines with high signature score are more likely to be p53 wild type (P <2.2x 10⁻¹⁶). See p53 mutation status for each cell line in Table 4. Furthermore, the majority of resistant cell lines with wild type TP53 but low signature score harbor mutations in key regulatory genes involved in MDM2-P53 interactions and downstream P53 pathways. This indicates that the signature score can serve as a surrogate mRNA-level indicator of MDM2-P53 pathway function.

**TABLE 4: p53 mutation status**

| Cell lines | Tissue | Origin | IC50 | Score | Mutation in TP53 |
|---|---|---|---|---|---|
| 10C9 | Lymphocyte B | Lymphoma, Non-Hodgkin's | 19.0 | -0.8 | Mutant |
| 143B | Bone | Osteosarcoma | 15.0 | 0.2 | Mutant |
| 22Rv1 | Prostate | Carcinoma | 1.0 | 3.0 | Mutant |
| 647-V | Urinary tract, urothelium | Carcinoma, urinary bladder, transitional cell carcinoma (TCC) | 17.0 | -1.6 | Mutant |
| 8305C | Thyroid gland | Carcinoma, medullary thyroid | 24.0 | -3.8 | Mutant |
| A-172 | Brain | Glioblastoma | 0.6 | 1.4 | Wild Type |
| A2058 | Skin | Melanoma | 17.0 | -2.8 | Mutant |
| A-375 | Skin | Melanoma, malignant | 0.3 | 2.8 | Wild Type |
| A549 | Lung | Carcinoma, squamous cell | 0.9 | 2.4 | Wild Type |
| A-673 | Muscle | Rhabdomyosarcoma | 18.0 | 0.9 | Mutant |
| ACHN | Kidney | Adenocarcinoma, renal cell | 0.3 | 4.1 | Wild Type |
| AGS | Stomach | Adenocarcinoma, gastric | 0.1 | 5.4 | Wild Type |
| AN3 CA | Uterus, endometrium | Adenocarcinoma | 18.0 | -1.2 | Mutant |
| ARH-77 | Lymphocyte B, peripheral blood | Leukemia, plasma cell | 17.0 | 0.3 | Mutant |
| AsPC-1 | Pancreas | Adenocarcinoma | 19.0 | -1.3 | Mutant |
| BFTC-905 | Urinary, bladder | Carcinoma, urinary bladder, transitional cell carcinoma (TCC) | 19.0 | -0.7 | Mutant |
| BT-474 | Breast, mammary gland | Carcinoma, ductal | 19.0 | 1.5 | Mutant |
| BT-483 | Breast, mammary gland | Carcinoma, ductal | 20.0 | 1.1 | Mutant |
| BxPC-3 | Pancreas | Adenocarcinoma | 18.0 | -2.2 | Mutant |
| C-33 A | Cervix | Carcinoma, cervix | 18.0 | -0.6 | Mutant |
| Caki-1 | Kidney | Carcinoma, clear cell | 0.4 | 4.5 | Wild Type |
| Calu-1 | Lung | Carcinoma, lung epidermoid OR Carcinoma, epidermoid pulmonary | 14.0 | -3.3 | Wild Type |
| Calu-3 | Lung | Adenocarcinoma | 11.0 | -3.4 | Mutant |
| Calu-6 | Lung | Carcinoma, anaplastic | 17.0 | -0.8 | Mutant |
| CAMA-1 | Breast, mammary gland | Adenocarcinoma, mammary gland, breast | 22.0 | 1.9 | Mutant |
| Capan-1 | Pancreas | Adenocarcinoma | 20.0 | 0.7 | Mutant |
| COLO 201 | Intestine, large; colon | Adenocarcinoma, colorectal | 10.0 | -3.2 | Mutant |
| COLO 320DM | Intestine, large; colon | Adenocarcinoma, colorectal | 12.0 | -4.9 | Mutant |
| COLO-704 | Ovary | Carcinoma, ovary | 20.0 | 0.5 | Wild Type |
| COLO-824 | Breast | Carcinoma | 15.0 | -2.9 | Wild Type |
| D341 Med | Brain, cerebellum | Medulloblastoma | 0.0 | 3.5 | Wild Type |
| DMS 114 | Lung | Carcinoma, classic small cell lung cancer | 10.0 | 1.7 | Mutant |
| DU145 | Prostate | Carcinoma, prostate | 1.0 | -4.1 | Mutant |
| EFO-21 | Ovary | Carcinoma, ovary | 31.0 | -2.1 | Mutant |
| F-36P | Myeloblast | Leukemia, acute myeloid | 23.0 | 2.7 | Mutant |
| HARA | Lung | Carcinoma, squamous cell | 19.0 | -5.1 | Mutant |
| HARA-B | Lung | Carcinoma, squamous cell | 10.0 | -4.1 | Mutant |
| HCC1143 | Breast, mammary gland | Carcinoma, primary ductal | 12.0 | 0.4 | Mutant |
| HCC1187 | Breast, mammary gland | Carcinoma, primary ductal | 12.0 | -3.7 | Mutant |
| HCC1395 | Breast, mammary gland | Carcinoma, primary ductal | 20.0 | -1.5 | Mutant |
| HCC1500 | Breast, mammary gland | Carcinoma, primary ductal | 1.0 | 4.8 | Wild Type |
| HCC1569 | Breast, mammary gland | Carcinoma, primary metaplastic | 15.0 | -2.1 | Mutant |
| HCC1806 | Breast, mammary gland | Carcinoma, primary acantholytic squamous cell | 11.0 | -4.2 | Mutant |
| HCC1937 | Breast | Carcinoma, ductal | 15.0 | -1.9 | NA |
| HCC1954 | Breast, mammary gland | Carcinoma, ductal | 12.0 | -4.2 | Mutant |
| HCC38 | Breast, mammary gland | Carcinoma, primary ductal | 12.0 | 0.5 | Mutant |
| HCT116 | Intestine, large; colon | Carcinoma, colorectal | 0.5 | 1.4 | Wild Type |
| HCT-8 | Intestine, large; colon | Adenocarcinoma, colorectal, ileocecal | 16.0 | 3.5 | Wild Type |
| HDLM-2 | Lymphocyte B | Lymphoma, Hodgkin's disease | 33.0 | 0.6 | Mutant |
| HEC-1-A | Uterus, endometrium | Adenocarcinoma | 16.0 | 0.0 | Mutant |
| HEL | Lymphocyte, peripheral blood | Erythroleukemia OR Leukemia, erythroid | 17.0 | 2.3 | Mutant |
| HeLa S3 | Cervix | Carcinoma, cervix | 20.0 | 0.7 | Mutant |
| HepG2 | Liver | Carcinoma, hepatocellular | 0.2 | 2.4 | Wild Type |
| HMCB | Skin | Melanoma | 15.0 | -2.2 | Mutant |
| HPAF-II | Pancreas | Adenocarcinoma | 10.0 | -0.6 | Mutant |
| Hs 38.T | Ovary | Teratoma | 0.7 | -0.4 | Wild Type |
| Hs 766T | Pancreas | Carcinoma | 17.0 | -0.7 | Mutant |
| HT-1080 | Connective tissue | Fibrosarcoma | 0.1 | 6.3 | Wild Type |
| HT-1197 | Urinary, bladder | Carcinoma, urinary bladder, papillary (PAP) | 0.2 | 6.5 | Wild Type |
| HT-1376 | Urinary, bladder | Carcinoma | 16.0 | 1.4 | Mutant |
| HuH-28 | Gall bladder | Carcinoma, bile duct OR Cholangiocarcinoma | 15.0 | -2.0 | Mutant |
| HUP-T4 | Pancreas | Carcinoma, pancreatic | 17.0 | -0.4 | Mutant |
| IM-9 | Lymphocyte B, peripheral blood | Myeloma, multiple | 0.1 | 4.2 | Wild Type |
| IMR-32 | Brain | Neuroblastoma (Glioma, neuroblastoma) | 0.4 | 3.2 | Wild Type |
| J82 | Urinary, bladder | Carcinoma, urinary bladder, transitional cell carcinoma (TCC) | 20.0 | -2.4 | Mutant |
| JIMT-1 | Breast | Carcinoma | 11.0 | -1.4 | Mutant |
| K-562 | Bone marrow | Leukemia, chronic myeloid | 16.0 | 5.5 | Mutant |
| KARPAS-299 | Blood | Lymphoma | 19.0 | 0.1 | Mutant |
| Kasumi-1 | Lymphocyte, peripheral blood | Leukemia, acute myeloid | 21.0 | -2.7 | Mutant |
| KELLY | Brain | Neuroblastoma (Glioma, neuroblastoma) | 15.0 | 0.0 | Mutant |
| KG-1 | Bone marrow | Leukemia | 15.0 | -2.5 | Mutant |
| KMM-1 | Lymphocyte | Myeloma | 19.0 | -0.6 | Mutant |
| KMS-11 | Lymphocyte | Myeloma, multiple | 12.0 | -1.7 | Wild Type |
| KMS-12-BM | Bone marrow | Myeloma, multiple | 0.0 | 1.3 | Mutant |
| KMS-20 | Lymphocyte | Myeloma | 13.0 | 0.0 | Mutant |
| KMS-21BM | Bone marrow | Myeloma, multiple | 0.0 | 3.6 | Wild Type |
| KMS-26 | Lymphocyte | Myeloma | 0.5 | -1.8 | Mutant |
| KYSE-520 | Esophagus; oesophagus | Carcinoma, squamous cell | 10.0 | -3.1 | Mutant |
| L-363 | Lymphocyte, peripheral blood | Leukemia, plasma cell | 32.0 | -0.6 | Mutant |
| LCLC-103H | Lung | Carcinoma, large cell, non-small cell lung cancer | 0.7 | -0.6 | Mutant |
| LN-18 | Brain, temporal lobe | Glioblastoma | 0.7 | 1.1 | Mutant |
| LNCaP clone FGC | NA | NA | 0.2 | 1.8 | Wild Type |
| LS 174T | Intestine, large; colon | Adenocarcinoma, colorectal | 0.6 | 3.6 | Wild Type |
| LS411N | Intestine, large; caecum | Carcinoma, colorectal | 16.0 | 0.9 | Mutant |
| LS513 | Intestine, large; caecum | Carcinoma, colorectal | 0.5 | 4.4 | Wild Type |
| MC/CAR | Lymphocyte B, peripheral blood | Myeloma, plasmacytoma | 0.7 | 3.3 | Wild Type |
| MCF7 | Breast, mammary gland | Adenocarcinoma, mammary gland, breast | 0.8 | 2.9 | Wild Type |
| MDA-MB-134-VI | Breast, mammary gland | Carcinoma, ductal | 38.0 | 0.4 | Mutant |
| MDA-MB-157 | Breast, mammary gland | Carcinoma, medula | 20.0 | -5.4 | Mutant |
| MDA-MB-231 | Breast, mammary gland | Adenocarcinoma | 17.0 | -1.2 | Mutant |
| MDA-MB-361 | Breast, mammary gland | Adenocarcinoma, mammary gland, breast | 21.0 | -0.6 | Mutant |
| MDA-MB-435S | Skin | Melanoma | 12.0 | -3.0 | Mutant |
| MDA-MB-453 | Breast, mammary gland | Carcinoma | 20.0 | -1.4 | Mutant |
| MDA-MB-468 | Breast, mammary gland | Adenocarcinoma, mammary gland, breast | 10.0 | -2.8 | Mutant |
| MEG-01 | null | Leukemia, chronic myeloid | 17.0 | -2.8 | Mutant |
| MFE-280 | Uterus, endometrium | Adenocarcinoma, malignant, endometroid carcinoma | 23.0 | -0.9 | Mutant |
| MFM-223 | Breast, mammary gland | Carcinoma, ductal | 17.0 | 2.9 | Mutant |
| MG-63 | Bone | Osteosarcoma | 16.0 | -1.0 | Mutant |
| MIA PaCa-2 | Pancreas | Carcinoma | 19.0 | -0.9 | Mutant |
| MKN-1 | Muscle, smooth, stomach | Carcinoma, squamous cell | 15.0 | -2.9 | Mutant |
| MKN-45 | Stomach | Adenocarcinoma | 0.2 | 4.7 | Mutant |
| MKN-74 | Muscle, smooth, stomach | Carcinoma, gastric | 0.9 | 6.8 | Wild Type |
| MOLM-13 | Blood | Leukemia, acute myeloid | 0.2 | 2.7 | Wild Type |
| MV-4-11 | Blood | Leukemia, acute monocytic | 0.0 | 2.4 | Wild Type |
| NCCIT | Germ cell, gametocyte | Teratocarcinoma | 11.0 | -1.3 | Mutant |
| NCI-H1155 | Lung | Carcinoma, non-small cell lung cancer | 22.0 | -1.6 | Mutant |
| NCI-H1299 | Lung | Carcinoma, non-small cell lung cancer | 15.0 | -2.5 | Mutant |
| NCI-H1437 | Lung | Adenocarcinoma, non-small cell lung cancer | 10.0 | -2.6 | Mutant |
| NCI-H1650 | Lung | Adenocarcinoma | 10.0 | 0.8 | Mutant |
| NCI-H1666 | Lung | Adenocarcinoma | 13.0 | 3.3 | Wild Type |
| NCI-H1793 | Lung | Adenocarcinoma, non-small cell lung cancer | 10.0 | 2.3 | Mutant |
| NCI-H187 | Lung | Carcinoma, classic small cell lung cancer | 15.0 | -1.7 | Mutant |
| NCI-H1975 | Lung | Adenocarcinoma | 16.0 | -2.6 | Mutant |
| NCI-H2081 | Lung | Carcinoma, small cell lung cancer | 17.0 | -2.2 | Wild Type |
| NCI-H209 | Lung | Carcinoma, small cell lung cancer | 11.0 | -1.6 | Mutant |
| NCI-H2122 | Lung | Adenocarcinoma | 0.2 | 2.8 | Mutant |
| NCI-H2170 | Lung | Carcinoma, squamous cell | 12.0 | 0.9 | Mutant |
| NCI-H2171 | Lung | Carcinoma, small cell lung cancer | 11.0 | -1.9 | Mutant |
| NCI-H226 | Lung | Carcinoma, squamous cell | 0.1 | 5.6 | Mutant |
| NCI-H23 | Lung | Adenocarcinoma, non-small cell lung cancer | 10.0 | -0.2 | NA |
| NCI-H28 | null | Mesothelioma | 0.5 | 4.3 | Wild Type |
| NCI-H345 | Lung | Carcinoma, small cell lung cancer | 17.0 | -1.9 | Mutant |
| NCI-H441 | Lung | Adenocarcinoma | 10.0 | -0.4 | Mutant |
| NCI-H446 | Lung | Carcinoma, small cell lung cancer | 18.0 | -0.9 | Mutant |
| NCI-H460 | Lung | Carcinoma | 0.9 | 1.9 | Wild Type |
| NCI-H520 | Lung | Carcinoma, squamous cell | 17.0 | -2.6 | Mutant |
| NCI-H522 | Lung | Adenocarcinoma, non-small cell lung cancer | 22.0 | -1.0 | Mutant |
| NCI-H526 | Lung | Carcinoma, small cell lung cancer | 16.0 | -2.7 | Mutant |
| NCI-H596 | Lung | Carcinoma, adenosquamous | 18.0 | -2.2 | Mutant |
| NCI-H661 | Lung | Carcinoma, large cell, neuroendocrine, non-small lung cancer | 11.0 | -2.0 | Mutant |
| NCI-H69 | Lung | Carcinoma, small cell lung cancer | 15.0 | -0.7 | Mutant |
| NCI-H716 | Intestine, large; caecum | Adenocarcinoma, colorectal | 13.0 | -1.9 | Mutant |
| NCI-H748 | Lung | Carcinoma, small cell lung cancer | 16.0 | 0.3 | Mutant |
| NCI-H838 | Lung | Adenocarcinoma, non-small cell lung cancer | 14.0 | 1.4 | Mutant |
| NCI-H929 | Lymphocyte B, bone marrow | Myeloma, plasmacytoma | 0.3 | 3.1 | Wild Type |
| NCI-N87 | Stomach | Carcinoma, gastric | 12.0 | -2.4 | Mutant |
| NIH:OVCAR-3 | Ovary | Adenocarcinoma | 17.0 | -3.6 | Mutant |
| NKM-1 | Lymphocyte | Leukemia, acute myeloid | 0.3 | 3.3 | Wild Type |
| NUGC-4 | Stomach | Adenocarcinoma | 0.4 | 4.8 | Wild Type |
| OPM-2 | Lymphocyte B | Myeloma, multiple | 13.0 | -2.1 | Mutant |
| PA-1 | Ovary | Carcinoma, ovary | 0.6 | 1.0 | Wild Type |
| PANC-1 | Pancreas | Carcinoma, epithelioid | 22.0 | 0.0 | Mutant |
| PC-10 | Lung | Carcinoma, squamous cell | 14.0 | -1.3 | Mutant |
| PC-13 | Lung | Adenocarcinoma | 14.0 | -1.7 | Mutant |
| PC-9 | Lung | Adenocarcinoma | 0.7 | 0.6 | Mutant |
| QG-56 | Lung | Carcinoma, non-small cell lung cancer | 10.0 | -4.7 | Mutant |
| RKO | Intestine, large; colon | Carcinoma, colorectal | 0.0 | 3.9 | Wild Type |
| RPMI-2650 | Nasal septum | Carcinoma, squamous cell | 0.8 | 3.9 | Wild Type |
| SBC-5 | Lung | Carcinoma, small cell lung cancer | 18.0 | -1.1 | Mutant |
| SCC-15 | Tongue | Carcinoma, squamous cell | 20.0 | -2.9 | Mutant |
| SCH | Stomach | Choriocarcinoma | 17.0 | -0.5 | Mutant |
| SH-SY5Y | Brain | Neuroblastoma (Glioma, neuroblastoma) | 0.8 | 2.6 | Wild Type |
| SJCRH30 | Muscle | Rhabdomyosarcoma | 16.0 | -4.1 | Mutant |
| SK-BR-3 | Pleura | Adenocarcinoma, mammary gland, breast | 21.0 | 0.4 | Mutant |
| SK-ES-1 | Bone | Sarcoma, Ewings | 25.0 | 0.1 | Mutant |
| SK-HEP-1 | Liver | Hepatoma, Hepatocellular carcinoma, HCC | 0.2 | 4.9 | Wild Type |
| SK-LU-1 | Lung | Adenocarcinoma | 11.0 | -0.7 | Mutant |
| SKM-1 | Lymphocyte, peripheral blood | Leukemia, acute myeloid | 19.0 | -2.7 | Mutant |
| SK-MEL-1 | Skin | Melanoma, malignant | 0.8 | 0.4 | Wild Type |
| SK-MEL-30 | Skin | Melanoma, malignant | 19.0 | -2.7 | Mutant |
| SK-N-AS | Brain | Neuroblastoma (Glioma, neuroblastoma) | 13.0 | -2.6 | Mutant |
| SK-N-F1 | Brain | Neuroblastoma (Glioma, neuroblastoma) | 17.0 | 0.4 | Mutant |
| SK-N-SH | Brain | Neuroblastoma (Glioma, neuroblastoma) | 0.2 | 2.4 | Wild Type |
| SK-OV-3 | Ovary | Adenocarcinoma | 17.0 | -3.3 | Mutant |
| SNU-1 | Stomach | Carcinoma, gastric | 0.4 | 3.6 | Wild Type |
| SNU-16 | Stomach | Carcinoma, gastric | 10.0 | -1.0 | Mutant |
| SNU-5 | Stomach | Carcinoma, gastric | 16.0 | -0.7 | Mutant |
| SR | Lymphocyte | Lymphoma, large cell | 0.0 | 3.9 | Wild Type |
| SW1116 | Intestine, large; colon | Adenocarcinoma, colorectal | 12.0 | -2.1 | Mutant |
| SW1463 | Intestine, large; rectum | Adenocarcinoma, colorectal | 1.0 | -1.5 | Mutant |
| SW480 | Intestine, large; colon | Adenocarcinoma, colorectal | 12.0 | -0.4 | Mutant |
| SW579 | Thyroid gland | Carcinoma, squamous cell | 16.0 | -0.8 | Mutant |
| SW780 | Urinary, bladder | Carcinoma, urinary bladder, transitional cell carcinoma (TCC) | 1.0 | 5.6 | Wild Type |
| SW837 | Intestine, large; rectum | Adenocarcinoma | 12.0 | -3.3 | Mutant |
| T24 | Urinary, bladder | Carcinoma, urinary bladder, transitional cell carcinoma (TCC) | 21.0 | -2.3 | Mutant |
| TCCSUP | Urinary, bladder | Carcinoma, urinary bladder, transitional cell carcinoma (TCC) | 16.0 | -3.3 | Mutant |
| TF-1a | Bone marrow | Erythroleukemia OR Leukemia, erythroid | 10.0 | -0.8 | Mutant |
| U-118 MG | Brain | Glioblastoma, astrocytoma | 19.0 | 0.4 | Mutant |
| U-138 MG | Brain | Glioblastoma | 29.0 | -1.6 | Mutant |
| U-2197 | Skin, hypodermis; subcutaneous | Histiocytoma, fibrous, malignant | 22.0 | -2.8 | Mutant |
| U266B1 | Lymphocyte B | Myeloma, plasmacytoma | 18.0 | 0.7 | Mutant |
| U-87 MG | Brain | Glioblastoma | 0.2 | 3.1 | Wild Type |
| UM-UC-3 | Urinary, bladder | Carcinoma, urinary bladder, transitional cell carcinoma (TCC) | 15.0 | -0.3 | Mutant |
| VCaP | Prostate | Carcinoma, prostate | 13.0 | -0.4 | Mutant |
| WERI-Rb-1 | Eye, retina | Retinoblastoma | 0.7 | 2.2 | Wild Type |
| Y79 | Eye, retina | Retinoblastoma | 0.6 | 4.9 | Wild Type |
| YAPC | Pancreas | Carcinoma, pancreatic | 18.0 | -1.5 | Mutant |
| ZR-75-1 | Breast, mammary gland | Carcinoma, ductal | 0.1 | 2.2 | Wild Type |

To examine the specific prediction power of the mRNA signature score across the studied tumor types, and to make sure the prediction from the signature score is not confounded by tumor lineage, the signature score within each available tumor type in CELLO is examined.

**TABLE 5: Correlation between the mRNA siganture score and mutation status of key regulatory genes invovled in MDM2-p53 interatctions and downstream p53 pathways**

| pathway | gene | proportion of mutant lines in resistant lines | propportion of mutant lines in sensitive lines | pvalue |
|---|---|---|---|---|
| apoptosis | ACINI | 68% | 58% | 1.32E-01 |
| apoptosis | TP73 | 5% | 0% | 1.33E-01 |
| apoptosis | SPTAN1 | 10% | 4% | 1.45E-01 |
| apoptosis | CASP6 | 4% | 0% | 1.77E-01 |
| apoptosis | IGFBP3 | 4% | 0% | 1.77E-01 |
| apoptosis | RFWD2 | 4% | 0% | 1.77E-01 |
| apoptosis | TP53INP1 | 67% | 58% | 1.80E-01 |
| apoptosis | AKT1 | 3% | 0% | 2.37E-01 |
| apoptosis | TP53I3 | 3% | 0% | 2.37E-01 |
| apoptosis | PTEN | 10% | 6% | 2.70E-01 |
| apoptosis | CASP7 | 2% | 0% | 3.24E-01 |
| apoptosis | NAIP | 2% | 0% | 3.24E-01 |

| | | mean score for mutant lines | mean score for wildtype lines | pvalue |
|---|---|---|---|---|
| apotosis | | -0.03 | 0.14 | 0.58 |
| cell cycle arrest | CDKN2A | 5% | 0% | 1.01E-01 |
| cell cycle arrest | RB1 | 14% | 6% | 1.01E-01 |
| cell cycle arrest | CCNB3 | 8% | 2% | 1.30E-01 |
| cell cycle arrest | CCNE1 | 2% | 0% | 3.24E-01 |

| | | mean score for mutant lines | mean score for wildtype lines | pvalue |
|---|---|---|---|---|
| cell cycle arrest | | -0.97 | 0.24 | 1.00E-02 |

To access the signature robustness measured under various technology platforms, the signature mRNA expressions and the composite score between RNA-seq qualification and microarray quantification is examined.

The performance of the MDM2 antagonist predictive mRNA response signature is tested in the clinical setting with specimens from the NO21279 leukemia trial (Figure 1). 28 AML patients treated at the MTD are enrolled and completed pretreatment and C1D10 (cycle 1, day 10) sampling. Patients are composed of 18 men and 10 women with a median age 59 years (Table 6). The clinical endpoint in NO21279 was divided into 4 categories: Complete Response (CR), Morphologic Leukemia-Free State (MLFS), Hematologic Improvement (HI), and Progressive Disease (PD). Blood leukemia samples and bone marrow biopsy samples were collected at baseline screening, after a single dose (cycle 1 day 2, C1D2) and on last day of dosing (cycle 1, day 10, C1D10) and isolated via MACS® separation ¹¹. The early pharmacodynamic effect of MDM2 antagonism in blood leukemia samples was assessed by measuring MDM2 RT PCR change between baseline and C1D10. Global gene expression profiles were generated for the peripheral blood leukemia cells and bone marrow biopsy samples obtained at baseline, C1D2 and C1D10.

The biomarker panel measurement procedures have been previously reported ¹¹. Analysis of TP53 mutations was done by Caris Life Sciences (Irving, TX, USA) using the PCR-based and microarray-based AmpliChip TP53 test (in development, Roche Molecular Systems, Pleasanton, CA, USA). This test reports single nucleotide substitutions or deletions in exons 2-11 and their splice sites^{11, 14}. MDM2 mRNA concentrations were assessed at Roche Molecular Systems by quantitative real-time PCR with 50 ng total RNA from MACs isolated leukemia cells from blood. TaqMan (Invitrogen, Carlsbad, CA, USA) probes were designed to detect MDM2 mRNA and the reference mRNA, beta-glucuronidase, simultaneously using two different fluorescent reporters. Gene expression profiles were generated using Affymetrix U133 Plus 2.0 microarrays.

Based on tumor specimen assessments, 23 of 28 patients have wild-type TP53 and 5 patients have TP53 mutations (Table 6). On day 1, median values for the area under the curve of 24hour (AUC0-24h) for RG7112 are 190,315 ng*h/mL (IQR: 119,032-242,857 ng*h/mL) among the 28 studied patients. Clinical responses in the 28 patients include 3 CR, 4 MLFS, 6 HI and 15 PD. Median MDM2 mRNA expression in samples from C1D10 is increased by 2.46 times (IQR: 1.62-4.59) over baseline, demonstrating a pharmacodynamic biomarker response resulting from p53 activation of MDM2 transcription. Drug exposure significantly correlates with patients' clinical responses (p=0.002). 8 of 15 PD patients have insufficient exposures, defined as AUC0-24h less than 150,000ng*h/mL; whereas only 1 patient has insufficient exposures in the other three categories.

**TABLE 6: Details of clinical trials**

| Trial | NO21279 | NO21280 | NP22890 |
|---|---|---|---|
| tumor type | AML | advanced malignancies | liposarcoma |
| no. of patients | 28 | 22 | 14 |
| Mean Age (SD) | 53.3 (17.6) | 57.1 (15.0) | 61.4 (14.5) |
| No. of Female (%) | 10 (35) | 11 (50) | 6 (43) |
| No. of patients with P53 mutation | 5 | 0 | 2 |

The identified 4-gene signature score is calculated for each of the 28 patients with AML by taking the summation of MDM2, BBC3, XPC, subtracting CDKN2A expression levels at baseline. There is a significant correlation between the signature scores and patients' clinical responses (PD<H1<MLFS<CR) to MDM2 antagonist therapy (Spearman correlation coefficient 0.58, P= 6.6x10⁻⁴) . The signature scores also significantly correlate with patients' pharmacodynamic biomarker responses as measured by MDM2 mRNA change from baseline to C1D10 (Spearman correlation coefficient 0.41, P= 0.02). The correlation between the signature scores and patients' clinical responses is further enhanced for the subset of 15 patients with sufficiently high exposures, defined as patients with AUC₀₋₂₄ₕ higher than 150,000ng*h/mL (Spearman correlation coefficient 0.64, P= 5.2x10⁻³).

This 4 gene signature panel is capable of distinguishing AML patients in response categories following treatment with MDM2 inhibitors in the following manner: CR/MLFS patients from PD/H1 patients with an AUC of the ROC curve of 0.82, and distinguishing CR/MLFS/H1 patients from PD patients with an AUC of 0.83. In contrast, MDM2 mRNA expression as a single biomarker could only distinguish CR/MLFS patients from PD/H1 patients with an AUC of 0.51, and distinguish CR/MLFS/H1 patients from PD patients with an AUC of 0.61. Using a cut-off point of the signature score 15, patients are classified into likely-responder group and likely-non-responder group at baseline prior to MDM2 antagonist therapy with 100% sensitivity and 71% specificity. Therefore, the signature panel has significant potential to be used as a companion predictive biomarker of MDM2 antagonist therapy to select a subset of AML patients who are most likely to respond; and avoid exposing the AML patients who are less likely to respond.

Therefore, disclosed herein is a method of identifying a patient suffering from cancer as likely to respond to a therapy comprising administration of an MDM2 inhibitor, the method comprising,
a) taking a sample from the patient;
b) measuring the expression levels of MDM2 mRNA in the sample;
c) comparing the MDM2 mRNA expression level from the patient to standard values from a patient with the same cancer; and
d) administering a compound which acts as inhibitor of the MDM2-p53 interaction.
The "standard value" is for example the cut-off value (cut-off point or reference level) for the patient signature score as defined herein.

In another embodiment, the present invention provides an *in vitro* method of identifying a patient suffering from cancer as likely to respond to a therapy comprising an MDM2 inhibitor, the method comprising,
a) measuring the mRNA expression level of MDM2, XPC, BBC3 and CDKN2A in a sample obtained from that patient prior to treatment;
b) applying the expression levels obtained in a) to a mathematical equation in order to calculate the patient's signature score;
c) comparing said patient's signature score obtained from b) to a reference level; and
d) identifiying said patient as more likely to respond to the therapy comprising said MDM2 inhibitor when the patients's signature score is above said reference level.
In one embodiment, the patient signature score above the reference level indicates a patient's high likelihood to respond to treatment with an MDM2 inhibitor, whereas a signature score below said level indicates that said patient is less likely to respond to that treatment.
In one embodiment, the sample obtained in a) is blood leukemia sample, or a bone marrow biopsy sample.
In another embodiment, the patient's signature score in b) is calculated from the sum of log2-transformed mRNA expression levels measured at baseline (i.e. prior to treatment), multiplied by the observed direction in-vitro, defined as signature score = G_{MDM2} + G_{XPC} + G_{BBC3} - G_{CDKN2A}. Within this embodiment, mRNA expression levels at baseline are measured by microarray measurements, preferably by using GeneChip Human Genome U133 Plus 2.0 Array. Also, within this embodiment, mRNA expression levels at baseline may be measured by RNA sequencing
In one embodiment, the reference level for the patient signature score as calculated using GeneChip Human Genome U133 Plus 2.0 Array measurement (the "microarray technology" or "microarray measurements") is 14.0 or 14.5. In another embodiment, the reference level for the patient signature score is 15. In a preferred embodiment, the reference level for the patient signature score is 15.4 (selected by the Youden's index).
In another embodiment, the patient signature socre is calculated based on mRNA expression levels obtained by RT PCR measurements. The patient signature score calculated using RT PCR may be different from the value calculated based on the microarray technology. However, within this embodiment according to the present invention, the patient signature score based on RT PCR can be converted into the value obtained when using microarray technology by established correlation between these methods, as well known to the person of skill in the art.
In another embodiment, when mRNA expression levels at baseline are measured by RT-PCR, the patient's signature score in b) is calculated from Algorithm 1: the sum of log2 transformed relative expression levels (delta-CP) measured at baseline (i.e. prior to treatment), multiplied by the observed direction in-vitro, defined as signature score = GMDM2 + GXPC + GBBC3 - GCDKN2A, where G = delta-CP, so delta-CPMDM2 = CPhousekeeper -CPMDM2, delta-CPXPC = CPhousekeeper -CPXPC, delta-CPBBC3 = CPhousekeeper -CPBBC3, delta-CPCDKN2A = CPhousekeeper -CPCDKN2A. The "housekeeper" gene is TMEM.
If measured on the same set of patient samples, the measurements of the signature score from RT-PCR (under the above Algorithm 1) is correlated with the measurements of the signature score from GeneChip Human Genome U133 Plus 2.0 Array with correlation coefficient 0.79. Within this embodiment, the reference level for the patient signature score from RT-PCR measurement is about 2.0, or 2.10 or 2.20. Still within this embodiment, the reference level for the patient signature score is 2.26 (selected by the Youden's index).
In yet another embodiment, when mRNA expression levels at baseline are measured by RT-PCR, the patient's signature score in b) can also be calculated from Algorithm 2: the sum of relative expression levels (2delta-CP) measured at baseline (i.e. prior to treatment), multiplied by the observed direction in-vitro, defined as signature score = GMDM2 + GXPC + GBBC3 - GCDKN2A, where G = 2delta-CP, delta-CP is defined in Algorithm 1. The "housekeeper" gene is TMEM.
If measured on the same set of patient samples, the measurements of the signature score from RT-PCR (under the above Algorithm 2) is correlated with the measurements of the signature score from GeneChip Human Genome U133 Plus 2.0 Array with correlation coefficient 0.63. Within this embodiment, the reference level for the patient signature score from RT-PCR measurement is about 4.10, or 4.20 or 4.30. Still within this embodiment, the reference level for the patient signature score is 4.34 (selected by the Youden's index).
In one embodiment the cancer is a haematological tumor, preferably AML, or MDS, or MPN.
In another embodiment, the cancer is a solid tumor, such as for example lung, prostate, colon, head, neck, or pancreatic cancer or sarcoma or melanoma.
An MDM2-inhibitor is a compound as specifically disclosed in U.S. Patent 8,354,444 B2, or in WO 2007/063013, WO 2010/031713, WO 2011/098398 and WO 2013/135648; or a compound according to formulae I, II, IIa or III, or combinations thereof, preferably of formulae IIa or III.
In one embodiment, the MDM2 inhibitor is the compound A (RG7112) as defined herein.
In another embodiment, the MDM2 inhibitor is the compound 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino }-3-methoxy-benzoic acid, of the formula 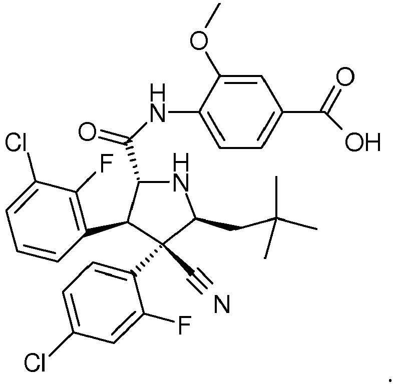 In yet another embodiment, the MDM2 inhibitor is the compound 4-{ [(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino }-3-methoxy-benzoic acid 1-mPEG-carbonyloxy-ethyl ester (mPEG, average MW, -2000), of the formula: 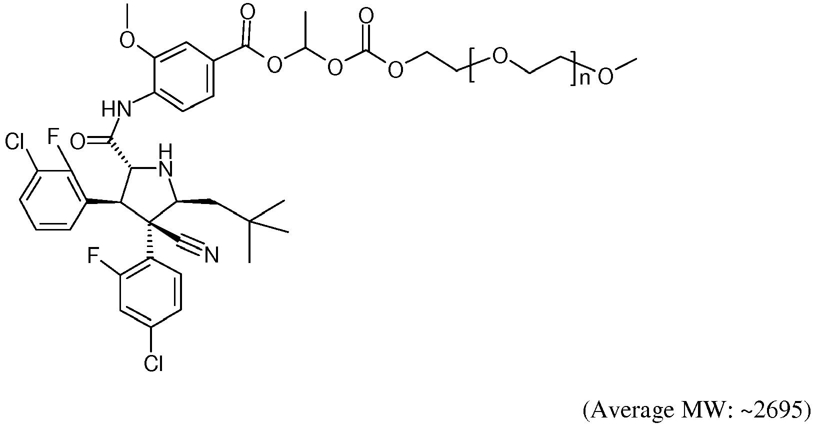 In yet another embodiment, the MDM2 inhibitor is the compound 4-{ [(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino }-3-methoxy-benzoic acid 1-mPEG-carbonyloxy-ethyl ester (mPEG, average MW, -2200): 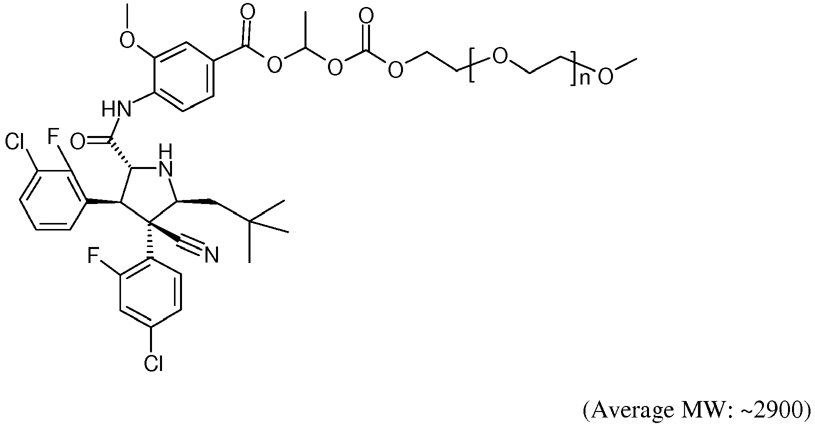 In still another embodiment, there is provided the use of a gene expression (mRNA) signature to predict a patients response to treatment with an MDM2 inhibitor (antagonist). Within this embodiment, the "gene expression signature" is a 4-gene mRNA signature consisting of MDM2, XPC, and BBC3 elevated expression as well as low expression of CDKN2A when measured at baseline, i.e. prior to treatment with an MDM2 inhibitor (antagonist).
The signature score measurements also correlate with patient's pharmacodynamic biomarker responses (MDM2 expression change). The data presented in Example 5 demonstrate that the 4 mRNA signature score according to the present invention is also a pharmacodynamic biomarker for monitoring efficacy of cancer treatment comprising an MDM2 inhibitor. Therefore, in another embodiment, there is provided an *in vitro* method for monitoring efficacy of therapy comprising an MDM2 inhibitor, as defined above, in a patient suffering from cancer, the method comprising
a) measuring the mRNA expression level of MDM2, XPC, BBC3 and CDKN2A in a sample obtained from that patient prior to treatment;
b) applying the expression levels obtained in a) to a mathematical equation in order to calculate the patient's signature score prior to treatment;
c) repeating step a) and b) after start of treatment with said MDM2 inhibitor; and
d) comparing the signature scores obtained after start of treatment with those obtained prior to treatment, whereas higher signature scores after treatment indicate a response of the patient to the treatment, and thus a recommendation to continue the treatment.
Within this embodiment, an MDM2 inhibitor is as defined above. The cancer is a solid tumor or AML. The sample obtained in a) is blood leukemia sample, or a bone marrow biopsy sample. Also within this embodiment, the signature score during treatment is preferably obtained at day 10 after the start of treatment, i.e. after the initial administration of an MDM2 inhibitor. The difference in the signature score at day 10 subsequent to initial dosing of an MDM2 inhibitor is at least 1.20 times the score measured at baseline, i.e. prior to treatment. In a preferred embodiment, a signature score at day 10 after initial dosing of an MDM2 inhibitor of about 1.23 to about 1.26 times the score measured prior to treatment, indicates that the patient responds to treatment and that the treatment should be continued.

A compound according to formula I, II, III, or combinations thereof, or pharmaceutically acceptable derivatives thereof may be used for the prophylactic or especially therapeutic treatment of the human or animal body (subject) (patient), in particular for treating a neoplastic disease (cancer). Examples of such cancers include, but are not limited to, epithelial neoplasms, squamous cell neoplasms, basal cell neoplasms, transitional cell papillomas and carcinomas, adenomas and adenocarcinomas, adnexal and skin appendage neoplasms, mucoepidermoid neoplasms, cystic neoplasms, mucinous and serous neoplasms, ducal-, lobular and medullary neoplasms, acinar cell neoplasms, complex epithelial neoplasms, specialized gonadal neoplasms, paragangliomas and glomus tumours, naevi and melanomas, soft tissue tumours and sarcomas, fibromatous neoplasms, myxomatous neoplasms, lipomatous neoplasms, myomatous neoplasms, complex mixed and stromal neoplasms, fibroepithelial neoplasms, synovial like neoplasms, mesothelial neoplasms, germ cell neoplasms, trophoblastic neoplasms, mesonephromas, blood vessel tumours, lymphatic vessel tumours, osseous and chondromatous neoplasms, giant cell tumours, miscellaneous bone tumours, odontogenic tumours, gliomas, neuroepitheliomatous neoplasms, meningiomas, nerve sheath tumours, granular cell tumours and alveolar soft part sarcomas, Hodgkin's and non-Hodgkin's lymphomas, other lymphoreticular neoplasms, plasma cell tumours, mast cell tumours, immunoproliferative diseases, leukemias, miscellaneous myeloproliferative disorders, lymphoproliferative disorders and myelodysplastic syndromes.

Particularly preferably, the disease according to the invention is a neoplastic disease, cancer, and more particularly AML.

Examples of cancers in terms of the organs and parts of the body affected include, but are not limited to, the breast, cervix, ovaries, colon, rectum, (including colon and rectum i.e. colorectal cancer), lung, (including small cell lung cancer, non-small cell lung cancer, large cell lung cancer and mesothelioma), bone, endocrine system, adrenal gland, thymus, liver, stomach, intestine, (including gastric cancer), pancreas, bone marrow, hematological malignancies, (such as lymphoma, leukemia, myeloma or lymphoid malignancies), bladder, urinary tract, kidneys, skin, thyroid, brain, head, neck, prostate and testis. Preferably the cancer is selected from the group consisting of breast cancer, prostate cancer, cervical cancer, ovarian cancer, gastric cancer, colorectal cancer, pancreatic cancer, liver cancer, brain cancer, neuroendocrine cancer, lung cancer, kidney cancer, hematological malignancies, melanoma and sarcomas. Especially preferably the cancer is selected from the group consisting of breast cancer, cervical cancer, ovarian cancer, colorectal cancer, melanoma and lung cancer. More especially preferably the cancer is selected from the group consisting of lung cancer, melanoma, ovarian cancer and colorectal cancer. In another preferred embodiment, for the case when the resistance predicted is acquired resistance, the cancer is lung cancer or ovarian cancer. In yet another preferred embodiment, for the case where the resistance predicted is inherent resistance, the cancer is selected from the group consisting of colorectal cancer, lung cancer or melanoma.

### Method of Treatment

In the context of the invention there is disclosed a method of treatment, wherein the activity level of a sample from a patient for sensitivity is first established relative to a standard level or set of standard levels or pre-treatment initiation levels and then an MDM2-inhibitor is administered. Preferably said MDM2-inhibitor is a compound of general formula I, II, III, or a pharmaceutically acceptable derivative thereof as defined above, more preferably a compound of formula IIa or III or a pharmaceutically acceptable derivative thereof. Said compounds may be administered in a pharmaceutical composition, as is well known to a person skilled in the art. Compositions for administration, such as nasal, buccal, rectal or, especially, oral administration, and for parenteral administration, such as intravenous, intramuscular or subcutaneous administration, to warm-blooded animals, especially humans, are especially preferred. More particularly, compositions for intravenous administration are preferred.

A compound of general formula I, II or III, preferably IIa or III, or a pharmaceutically acceptable derivative thereof can be administered alone or in combination with one or more other therapeutic agents. Possible combination therapy may take the form of fixed combinations, or the administration of a compound of the invention and one or more other therapeutic agents which are staggered or given independently of one another, or the combined administration of fixed combinations and one or more other therapeutic agents.

A compound of general formal I, II or III, preferably IIa or III, or a pharmaceutically acceptable derivative thereof can, besides or in addition, be administered especially for tumour therapy in combination with chemotherapy (cytotoxic therapy), targeted therapy, endocrine therapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumour regression, or even chemo-preventive therapy for example in patients at risk.

### Kit and Device

In one aspect disclosed herein is a kit and in another aspect a device for predicting the response, preferably of a cancer in a subject (or patient), to a compound of general formula I, II, III, preferably IIa or III, or a pharmaceutically acceptable derivative thereof as defined, comprising reagents necessary for measuring the MDM2 gene.

The kit and device may also preferably comprise a comparator module which comprises a standard value or set of standard values to which the level of MDM2 in the sample is compared. In a preferred embodiment, the comparator module is included in instructions for use of the kit. In another preferred embodiment the comparator module is in the form a display device, for example a strip of colour or numerically coded material which is designed to be placed next to the readout of the sample measurement to indicate resistance levels. The standard value or set of standard values may be determined as described above.

The following examples are illustrative of the invention and not limitative thereof.

### EXAMPLES

### EXAMPLE 1

### RNA sequencing of CELLO cell lines

RNA sequencing (RNA-seq) by Next Generation Sequencing (NGS) technology is an accurate and sensitive approach to measure gene expression, with the additional power to detect alternative splicing, allele specific expression, non-coding RNA, and various forms of mutations (SNPs, indels, gene fusions). The NGS Illumina HiSeq machine generates raw base calls in reads of 50 or 100 bp length, which are subjected to several data analysis steps. The RNA-seq is conducted at 40 to 50 million reads per sample. This number provides relatively high sensitivity to detect low-expressed genes while allowing for cost-effective multiplexing of samples. RNA is prepared by standard kits and RNA libraries by polyA TruSeq Illumina kits. 100 ng of mRNA per cell line is used for each RNA-seq reaction. A number of quality control procedures are applied to the RNA-seq data for each sample. The Illumina HiSeq software reports the total number of clusters (DNA fragments) loaded in each lane, percent passing sequencing quality filters (which identifies errors due to overloading and sequencing chemistry), a phred quality score for each base of each sequence read, overall average phred scores for each sequencing cycle, and overall percent error (based on alignment to the reference genome). For each RNA-seq sample, the percentage of reads that contain mitochondrial and ribosomal RNA is calculated. The FASTQC package is used to provide additional QC metrics (base distribution, sequence duplication, overrepresented sequences, and enriched kmers) and a graphical summary. Finally, the Picard toolkit that provides additional RNA-seq metrics including an estimate of 3" bias is used (caused by the use of poly-A capture or priming for cDNA synthesis in sample preparation), the percentage of reads mapping to exons, introns and intergenic regions.

### EXAMPLE 2

### Exom-Sequencing of CELLO cell lines

### COMPOUND A in vitro Assay

281 cells are treated with MDM2 antagonists and the IC50 (therapy response) is generated. The cell lines span a broad range of tumor cell types of derivation.

Among the 281 cell lines evaluated, 210 cell lines show mutations in TP53 after a careful annotation removing low quality calls and germline mutations. Among the 7 AML cell lines tested, 5 showed mutations in TP53. Cell lines harboring mutant TP53 are much less sensitive to MDM2 antagonist therapy (P<2.2x10⁻¹⁶), consistent with previously published data. The genome-wide association between baseline mRNA expression and MDM2 antagonist therapy response (IC50) identify a list of 13 genes with significant associations with P-values ranging from 2.38x10⁻⁴⁷ to 9.56x10⁻²³ (Supplementary Table 2). Functional annotation indicated that the 13 significant genes from the genome-wide association, with correlation coefficients ranging from -0.47 to -0.31 (with one positive correlation 0.28), are known regulators in the relevant MDM2-P53 interactions or downstream P53 pathways, including cell cycle arrest and apoptosis (Supplementary Table 2). Among them, MDM2 has the 4^{th} highest gene with an over-expression of MDM2 correlating with in vitro sensitivity, consistent with previously published data.

A multivariate logistic regression classifier is identified, which contains up-regulations of three genes including MDM2, XPC (xeroderma pigmentosum, complementation group C), BBC3 (BCL2 binding component 3) and down-regulation of tumor suppressor gene CDKN2A (cyclin-dependent kinase inhibitor 2A) (Table 2). The final signature is capable of distinguishing MDM2 antagonist sensitive cell lines from MDM2 antagonist resistant cell lines with AUC of 0.93 (95% CI 0.92 to 0.95). Therefore, the MDM2 antagonist sensitive cell lines demonstrate baseline up-regulation of MDM2, XPC, and BBC3 and down-regulation of CDKN2A; whereas the MDM2 antagonist resistant cell lines are characterized by down-regulation of MDM2, XPC, and BBC3 and up-regulation of CDKN2A (Figure 2).

**TABLE 7: Biosignature genes performance in CELLO and in NP21279**

| | CELLO | | | | NO21279 | | | |
|---|---|---|---|---|---|---|---|---|
| | OR (95% CI)^{a} | *P*-value^{b} | Correlation Coefficient RNAseq results vs microarray results | *P*-value | Correlation (efficacy) | *P*-value | Correlation (change in MDM2) | *P*-value |
| MDM2 | 4.17 (2.12, 8.19) | 3.41E-05 | 0.73 | 3.50E-19 | 0.14 | 2.32E-01 | 0.22 | 1.49E-01 |
| XPC | 3.42 (1.80, 6.49) | 1.78E-04 | 0.63 | 1.72E-16 | 0.27 | 8.58E-02 | 0.49 | 7.40E-03 |
| BBC3 | 1.62 (0.95, 2.78) | 7.67E-02 | 0.38 | 1.20E-14 | 0.37 | 2.68E-02 | 0.05 | 4.09E-01 |
| CDKN2A | 0.48 (0.29, 0.79) | 4.41E-03 | 0.89 | 2.46E-26 | -0.23 | 1.20E-01 | -0.29 | 7.62E-02 |
| score | 2.53 (1.95, 3.29) | 3.52E-12 | 0.8 | 1.30E-21 | 0.58 | 9.00E-04 | 0.41 | 1.90E-02 |

In addition to the target gene MDM2, the other three genes in the signature are all biologically supported as regulators in the MDM2-p53 interactions or downstream p53 pathways. The XPC gene plays an important role involved in repairing damaged DNA, contributing to damage recognition, open complex formation, and repair protein complex formation. BBC3, also known as p53 upregulated modulator of apoptosis (PUMA), were induced by exposure to DNA-damaging agents and by p53, which mediates DNA damage-induced apoptosis. The two gene products of the CDKN2A, p16 and p14ARF, are both linked to major tumor suppressor pathways; especially p14ARF, which inhibits MDM2 function by sequestering it in the nucleolus. To examine the molecular mechanisms underlying the mRNA signature, we further correlate the mRNA signature score with mutation status of p53 and key regulatory genes involved in MDM2-p53 interactions and downstream p53 pathways (Table 4). As shown in Figure 2, cell lines with low signature score were more likely to be p53 mutant; whereas cell lines with high signature score are more likely to be p53 wild type (P < 2.2x10⁻¹⁶). Furthermore, we identified that the majority of resistant cell lines with wild type TP53 but low signature score harbor mutations in key regulatory genes involved in MDM2-p53 interactions and downstream p53 pathway. These evidences indicated that the signature score can potentially serve as a surrogate mRNA-level indicator of MDM2-P53 pathway function.

To examine the specific prediction power of the mRNA signature score across the studied tumor types, and to make sure the prediction from the signature score is not confounded by tumor lineage, further examine the signature score within each available tumor type in CELLO (Table 8) is examined. Of note, the predictive power of the signature score is preserved in the AML cell lines.

**TABLE 8: Tumor type specific correlation**

| Tissue | Number | % Sen | % Res | Mean(Sen) | SD(Sen) | Mean(Res) | SD(Res) | Cor | Pvalue |
|---|---|---|---|---|---|---|---|---|---|
| Bone marrow | 5 | 40.0 | 60.0 | 2.5 | 1.6 | 0.7 | 4.2 | 0.1 | 0.0 |
| Brain | 10 | 50.0 | 50.0 | 2.6 | 0.7 | -0.7 | 1.3 | -0.8 | 0.0 |
| Breast, mammary gland | 20 | 15.0 | 85.0 | 3.3 | 1.3 | -1.1 | 2.4 | -0.1 | 0.0 |
| Intestine, large; colon | 8 | 37.5 | 62.5 | 3.0 | 1.4 | -1.4 | 3.2 | -0.5 | 0.0 |
| Lung | 41 | 14.6 | 85.4 | 2.1 | 2.1 | -1.3 | 1.9 | -0.5 | 0.0 |
| Lymphocyte | 6 | 50.0 | 50.0 | 1.8 | 3.1 | -0.8 | 0.9 | -0.5 | 0.0 |
| Ovary | 6 | 33.3 | 66.7 | 0.3 | 0.9 | -2.1 | 1.9 | -0.3 | 0.0 |
| Pancreas | 9 | 0.0 | 100.0 | NA | NA | -0.8 | 0.9 | 0.2 | 0.0 |
| Skin | 6 | 33.3 | 66.7 | 1.6 | 1.7 | -2.7 | 0.3 | -0.6 | 0.0 |
| Stomach | 8 | 50.0 | 50.0 | 4.6 | 0.8 | -1.2 | 0.8 | -0.7 | 0.0 |
| Urinary, bladder | 8 | 25.0 | 75.0 | 6.0 | 0.6 | -1.3 | 1.7 | -0.8 | 0.0 |

A robust demonstration of utility of an mRNA signature across various assayplatforms is critical in clinical in development of a biomarker. In the case of this study, the signature is developed with RNAseq quantification of expression measurement, which takes advantage of its sensitivity and the increased dynamic range over microarray technology. As shown in Table 2, the mRNA expression levels of the four genes measured through RNAseq are well correlated with those measured through microarrays. The high discriminatory ability of individual genes and the overall score are preserved through microarray quantification.

### EXAMPLE 3

### In vitro Assay Gene Expression Analysis Methods

Messenger RNA (mRNA) expression levels at baseline, prior to MDM2 antagonist therapy, are obtained via RNA sequencing (RNA-seq) and microarray measurement using GeneChip Human Genome U133 Plus 2.0 Array. Gene expression analysis of RNA-seq data is summarized here. First, the sequence reads are mapped to the reference human genome and to an additional database of splice junction fragments derived from known exon locations on the reference genome (Cufflinks software). These mapped reads are then combined to create discrete counts of reads (or sequenced bases) per gene. These gene expression counts are then normalized to equalize the total amount of RNA counts for each sample and corrected for gene/transcript length (RPKM). All genes with expression values less than RPKM = 1 in all cell lines are removed (this is roughly equivalent to an expression level less than one copy of an RNA molecule per cell). Next, normalized gene counts are statistically tested to identify differentially expressed genes between responder cell lines and non-responder cell lines using statistical methods that find differentially expressed (DE) genes with negative binomial models. The negative binomial model implemented is the DEseq software. 20355 genes are subject to the differential expression analysis after QC. Bonferroni correction threshold was used to determine the statistical significance. False discovery rate is also estimated and reported.

### Development of 4-gene signature

The 12 significant genes from univariate DE analysis are set as candidates for building the signature. The genes are ranked via their fold changes of responder cell lines to non-responder cell lines. Positive genes are defined as those with fold changes higher than 1; whereas negative genes are defined as those with fold changes less than 1. A multivariate logistic regression classifier is then built through a upward model selection procedure to maximize area under the receiver operating characteristic (ROC) curve with 10-fold cross-validation. The mode selection is implemented procedure through R package bestglm. The final selected model is composed of 4 genes. Therefore, each cell line is estimated by a linear combination of their gene-expression values (log transformed RPKM) weighted by their regression coefficients, defined as COMPOUND A signature = 1.43G_{MDM2} + 1.23G_{XPC} + 0.48G_{BBC3} - 0.73G_{CDKN2A}

### Microarray Gene Expression Analysis Methods

To reduce variation among microarrays, the intensity values for samples in each microarray are rescaled by quantile normalization method. Each intensity value is then log2 transformed.

### Evaluation of the 4-gene signature in COMPOUND A clinical trials

In the NO21279 Phase 1 clinical trial, patients with relapsed/refractory leukemia are treated with ascending doses of COMPOUND A. Specimens from subset of patients treated at the MTD (1500 mg BID x 10 days) are evaluated as a part of the current gene expression study. Blood leukemia samples are collected at baseline, cycle 1 day 2 (C1D2) and cycle 1, day 10 (C1D10) and isolated via MACs separation.

Risk scores are calculated for patients based on their mRNA expression values of the 4 genes at baseline prior to treatment. To ensure a rigorous and an unbiased validation, the coefficients in the test cohort is not applied. However, due to the platform difference and the profound biological differences between patients and cell lines, the set of in-vitro coefficients for the patient level classifier cannot be directly applied. Therefore, the patient's signature scores is simply the sum of expression levels of the signature genes as measured by microarray analysis, multiplied by the observed direction in-vitro, defined as COMPOUND A signature = G_{MDM2} + G_{XPC} + G_{BBC3} - G_{CDKN2A}. Note that it is not an optimal combination of the gene expression levels. So, the reported association and prediction power of the score is a conservative estimate of the score performance.

### EXAMPLE 4

### Microarray Gene Expression Analysis Methods

To reduce variation among microarrays, the intensity values for samples in each microarray are rescaled by quantile normalization method. Each intensity value is then log2 transformed.

### Validation of the 4-gene signature in COMPOUND A clinical trials

In the NO21279 Phase 1 clinical trial, patients with relapsed/refractory leukemia are treated with ascending doses of COMPOUND A. Specimens from subset of patients treated at the MTD (1500 mg BID x 10 days) are evaluated as a part of the current gene expression study. Leukemia samples are collected at baseline, cycle 1 day 2 (C1D2) and cycle 1, day 10 (C1D10) and isolated via MACs separation.

Risk scores are calculated for patients based on their mRNA expression values of the 4 genes at baseline prior to treatment. To ensure a rigorous and an unbiased validation, the compute of the coefficients in the test cohort is not done. However, due to the platform difference and the biological differences between patients and cell lines, in-vitro coefficients for the patient level classifier is not applied. Therefore, the patient's signature scores is simply the sum of expression levels of the signature genes as measured by microarray analysis, multiplied by the observed direction in-vitro, defined as COMPOUND A signature = G_{MDM2} + G_{XPC} + G_{BBC3} - G_{CDKN2A}. Note that it is not an optimal combination of the gene expression levels, so the reported association and prediction power of the score is a conservative estimate of the score performance.

### EXAMPLE 5

### In Vivo Testing

In the NO21279 Phase 1 clinical trial, patients with relapsed/refractory leukemia are treated with ascending doses of RG7112. Specimens from subset of patients treated at the MTD (1500 mg BID x 10 days) are evaluated as a part of the current gene expression study. Leukemia samples are collected at baseline, cycle 1 day 2 (C1D2) and cycle 1, day 10 (C1D10) and isolated via MACs separation.

Patients are composed of 18 men and 10 women with a median age 59 years. Based on tumor specimen assessments, 23 of 28 patients have wild-type TP53, and 5 patients have TP53 mutations (one patient with mutation A to C in intron 7; two patients with mutations CGC to CAC in exon 5; one patient with deletion G 323_3-324_1 in exon 9 farmeshift; one with mutation S240G -- S240, AGT to GGT in exon 7). On day 1, median values for the area under the curve of 24 h (AUC0-24h) for COMPOUND A are 190,315 ng*h/mL (IQR: 119,032-242,857 ng*h/mL) among the 28 studied patients. Clinical responses are evaluated and are divided into 4 categories: Complete Response (CR), Morphologic Leukemia-Free State (MLFS), Hematologic Improvement (HI), and Progressive Disease (PD). Response are evaluated in the 28 patients including 3 CR, 4 MLFS, 6 HI and 15 PD. In addition to assessments of baseline samples, treatment samples are also evaluated (Cycle 1, Day 10). Median MDM2 mRNA expression in biopsies from C1D10 is increased by 2.46 times (IQR: 1.62-4.59) over baseline, demonstrating a pharmacodynamic biomarker response resulting from p53 activation of MDM2 transcription.

Baseline blood cell specimens from 28 evaluable patients dosed at the MTD are evaluated using Affymetrix GeneChip Human Genome U133 Plus 2.0 microarrays. The identified 4-gene signature score is calculated for each of the 28 patients by taking the summation of MDM2, BBC3, XPC, subtracting CDKN2A expression levels at baseline. There is a significant correlation between the signature scores and patients' clinical responses (PD<HI<MLFS<CR) to MDM2 antagonist therapy (Spearman correlation coefficient 0.58, P= 6.6x10⁻⁴). The signature scores also significantly correlate with patients' pharmacodynamic biomarker responses as measured by MDM2 mRNA change from baseline to C1D10 (Spearman correlation coefficient 0.41, P= 0.02; Figure 3). The correlation between the signature scores and patients' clinical responses is further enhanced for the subset of 15 patients with sufficiently high exposures, defined as patients with AUC0-24h higher than 150,000ng*h/mL (Spearman correlation coefficient 0.64, P= 5.2x10⁻³). This panel was capable of distinguishing CR/MLFS patients from PD/HI patients with an AUC of the ROC curve of 0.82 (Figure 4, Table 10), and distinguishing CR/MLFS/HI patients from PD patients with an AUC of 0.83 (Figure 4). In contrast, MDM2 mRNA expression as a single biomarker could only distinguish CR/MLFS patients from PD/HI patients with an AUC of 0.51, and distinguish CR/MLFS/HI patients from PD patients with an AUC of 0.61.

| **Table 10.** Assessment of the MDM2-antagonist therapy predictive signature | | | | | |
|---|---|---|---|---|---|
| Patient no. | Response | TP53 Status | AUC₀₋₂₄ (ng*h/mL) | MDM2 mRNA (RT-PCR) fold change over baseline (C1D10) | Patient's signature score measured from microarray technology (and from RT-PCR calculated from algorithm 1) at baseline |
| 1 | CR | WILD-TYPE | 242,190 | 1.67 | 16.53 (3.52) |
| 2 | CR | WILD-TYPE | 234,520 | 3.00 | 16.01 (2.83) |
| 3 | CR | WILD-TYPE | 139,330 | 2.23 | 15.37 (3.50) |
| 4 | MLFS | WILD-TYPE | 268,300 | 5.67 | 16.18 (3.93) |
| 5 | MLFS | WILD-TYPE | 247,574 | 5.05 | 15.68 (3.04) |
| 6 | MLFS | MUTANT | 330,300 | 9.66 | 15.28 (2.26) |
| 7 | MLFS | WILD-TYPE | 189,500 | 1.79 | 15.23 (2.30) |
| 8 | HI | WILD-TYPE | 239,490 | 23.00 | 16.49 (3.91) |
| 9 | HI | WILD-TYPE | 244,860 | 2.01 | 15.93 (2.91) |
| 10 | HI | WILD-TYPE | 191,130 | 1.72 | 15.75 (3.92) |
| 11 | HI | WILD-TYPE | 400,900 | 5.07 | 14.92 (1.97) |
| 12 | HI | WILD-TYPE | 209,420 | 3.20 | 14.66 (1.57) |
| 13 | HI | WILD-TYPE | 185,630 | 4.35 | 13.95 (2.36) |
| 14 | PD | WILD-TYPE | 65,080 | 1.27 | 16.36 (3.41) |
| 15 | PD | MUTANT | NA | NA | 15.47 (3.28) |
| 16 | PD | WILD-TYPE | 124,790 | 2.51 | 15.46 (1.25) |
| 17 | PD | WILD-TYPE | 83,480 | 0.96 | 15.27 (2.10) |
| 18 | PD | WILD-TYPE | NA | NA | 15.14 (0.37) |
| 19 | PD | WILD-TYPE | 127,530 | 1.69 | 15.00 (2.68) |
| 20 | PD | WILD-TYPE | NA | NA | 14.99 (1.86) |
| 21 | PD | WILD-TYPE | 81,720 | 2.26 | 14.86 (1.58) |
| 22 | PD | WILD-TYPE | 101,760 | 1.52 | 14.84 (0.98) |
| 23 | PD | MUTANT | 207,580 | 3.88 | 14.83 (1.78) |
| 24 | PD | WILD-TYPE | NA | 2.65 | 14.68 (NA) |
| 25 | PD | WILD-TYPE | 396,800 | 5.11 | 14.07 (-0.99) |
| 26 | PD | WILD-TYPE | 159,120 | 9.46 | 13.90 (NA) |
| 27 | PD | MUTANT | 89,740 | 1.00 | 12.93 (-1.19) |
| 28 | PD | MUTANT | 78,630 | 1.59 | 12.41 (-0.79) |
| All patients | 3CR, 4 MLFS, 6 HI, 15 PD | 23 WT, 5 Mutant | 190,325^{a} (IQR: 119,032-242,857) | Median increase 2.46x (IQR: 1.62-4.59) | Average WT score 15.54 ± 0.16 (2.33±0.28) Average mutant score 14.46 ± 0.63 (1.23±0.73) |

| | | | | | |
|---|---|---|---|---|---|
| ^{a} The median exposure value for the area under the curve of 24 hour (AUC₀₋₂₄ₕ) for RG7112 among the 28 studied patients on Day 1. ^{b} AUC₀₋₂₄ₕ less than 150,000 ng*h/mL defined as insufficient exposures | | | | | |

Using a cut-off point of the signature score 15, or 15.4 (selected by Youden's Index) patients are classified into likely-responder group and likely-non-responder group at baseline prior to MDM2 antagonist therapy with 100% sensitivity and 71% specificity. Therefore, the signature panel has significant potential to be used as a companion predictive biomarker of MDM2 antagonist therapy to select a subset of AML patients who are most likely to respond; and avoid exposing the AML patients who are less likely to respond.

To understand the molecular mechanisms underlying the mRNA signature, the mRNA siganture score is correlated with mutation status of TP53. Patients with low signature scores are more likely to be TP53 mutants; whereas patients with high signature score are more likely to be TP53 wild type or TP53 mutant with predicted benign mutations (P = 6.86 x 10-2). Among the five p53 mutated patinets, four show progressive disseases with average score 14.2±0.8 (one patient with mutation A to C in intron 7; 2 patients with mutations CGC to CAC in exon 5; one patient with deletion G 323_3-324_1 in exon 9 frameshift), while one shows MLFS with mutation S240G - S240, AGT to GGT in exon 7, predicts not direct interact with DNA or with any amino acid residues that interact with DNA by IARC database, and score 15.8. This evidence indicates that the signature socre can potentiallys erve as a surrogate mRNA-level indicator of MDM2-P53 pathway function.

To examine the tissue specificity of the mRNA signature, baseline bone marrow cell specimens are measured from the available subset of 18 patients dosed at the MTD using Affymetrix GeneChip Human Genome U133 Plus 2.0 microarrays. The blood-signature score and bone-marrow-signature score are significantly correlated with Spearman correlation coefficient 0.50 (P = 0.016). The bone-marrow-signature scores were significantly correlated with patients' clinical responses and pharmacodynamic biomarker responses (MDM2 expression change from cycle 1 day 1 to cycle 1 day 10) to MDM2 antagonist therapy with Spearman correlation coefficients 0.46 (P = 0.052) and 0.42 (P = 0.069) respectively.

To study the pharmacodynamics properties of the mRNA signature, blood cell specimens are measured from most of the 28 patients on C1D10 and derived the signature on C1D10. Median MDM2, XPC and BBC3 mRNA expression in samples from C1D10 is 2.37 times (IQR: 1.71-5.00), 1.69 times (IQR: 1.27-1.88), 1.45 times (IQR: 1.13-1.99) over baseline, demonstrating up-regulation of respective genes stimulated by MDM2 antagonist therapy for the positively correlated genes in the signature. On the other hand, median CDKN2A mRNA expression in biopsies from C1D10 is decreased by 0.26 times (IQR: 0.09-0.38) over baseline, demonstrating a down-regulation stimulated by MDM2 antagonist therapy for the negatively correlated gene in the signature. Therefore, the overall signature score in biopsies from C1D10 is 3.05 times (IQR: 1.88-4.23) over baseline, demonstrating an up-regulation of the overall score stimulated by MDM2 antagonist therapy. In summary, the signature scores are correlated with patients' clinical responses consistently when measured at baseline (correlation coefficient 0.58, P= 9x10-4), and during the treatment regimen: C1D2 (correlation coefficient 0.40, P= 2.69x10-2), and C1D10 (correlation coefficient 0.65, P=3.5x10-4). The signature score measurements are also correlated with patients' pharmacodynamic biomarker responses (MDM2 expression change) consistently when measured at baseline (correlation coefficient 0.41, P= 1.92x10-2), C1D2 (correlation coefficient 0.64, P= 1.07x10-3), and C1D10 (correlation coefficient 0.64, P= 3.3x10-4). Median signature score on C1D2 is 1.11 times (IQR: 1.05-1.16), 1.08 times (IQR: 1.04-1.10), 1.17 times (IQR: 1.14-1.19), and 1.15 times (IQR: 1.13-1.15) over baseline for PD, HI, MLFS and CR patients respectively. Median score on C1D10 is 1.14 times (IQR: 1.08-1.21), 1.25 times (IQR: 1.23-1.30), 1.26 times (IQR: 1.23-1.31), and 1.23 times (IQR: 1.22-1.24) over baseline for PD, HI, MLFS and CR patients respectively. This evidence indicates that the mRNA signature score is both a predictive biomarker and a pharmacodynamics biomarker of MDM2 antagonist therapyactivity. The baseline mRNA signature score is thus predictive of patients' responses to MDM2 inhibitor, and MDM2 antagonist therapy stimulates patients by various extents during the treatment cycle that is indicative of the patients' clinical responses (Figure 3).

### EXAMPLE 6

### Assessment of MDM2 antagonist therapy predictive siganture in Phase I Solid Tumors trials

The predictive mRNA signature is also evaluated in two solid tumor trials (Figure 1). In trial NP21280 30 patients with pretreatment and C1D5 tumor biopsy samples are evaluated; and in NP22890 20 patients with pretreatment and C1D8 tumor biopsy samples are evalauted (Table 1). In both clinical trials, a biomarker package that enabled assessment of p53 pathway activation is assessed (p53 IHC, p21 IHC, MDM2 mRNA, and Ki67), MDM2 inhibition activates the P53 pathway and decreases cell proliferation. As previously reported, P53 and P21 concentrations, and MDM2 mRNA expression all significantly increase at C1D8 from baseline, and Ki-67-positive tumor cells are decreased from baseline in NP22890. These changes in biomarker responses are not significantly correlated with drug exposure, except for MIC-1 change12. In NP21280, P53 and P21 concentrations, and MDM2 mRNA expressions, all significantly increase at C1D10 from baseline (p=2.88x10-3, 2.32x10-3, 1.03x10-5, respectively); and Ki-67-positive tumor cells are decreased from baseline (p=2.62x10-2). These changes in biomarker responses are also significantly correlated with drug exposure (p=4.77x10-3 for P53 change, p=5.30x10-2 for P21 change, and p=2.5 x10-3 for MDM2 change), except for the number of Ki-67-positive tumor cells.

The signature scores are similarly derived for patients at baseline and at C1D5 (NO21280) and C1D8 (NP22890). The mRNA signature scores show heterogeneous magnitudes of the correlations with the COMPOUND A pharmaco-kinetics and pharmacodynamic marker panels and between the two trials. In NP21280, the baseline score significantly correlates with patients' MDM2 mRNA expression change with correlation coefficient 0.41(p=2.82x10-2), but no significant correlation between the signature score and P21 or the number of Ki-67-positive tumor cells is observed. In NP22890, only a suggestion of a positive correlation between the signature score and MDM2 change and P21 change are observed (Table 9). No assessment of clinical outcomes is feasible for the solid tumor trial specimens.

**TABLE 9: Correlation between score and biomarker response in NO21280 and NP22890**

| | NO21280 | | NP22890 | |
|---|---|---|---|---|
| | Correlation coefficient | P-value | Correlation coefficient | P-value |
| Ki-67 Change | -0.20 | 2.25E-01 | 0.35 | 8.82E-01 |
| P53 Change | 0.06 | 4.15E-01 | -0.06 | 5.76E-01 |
| P21 Change | 0.17 | 2.85E-01 | 0.16 | 3.01E-01 |
| MDM2 Change | 0.41 | 2.82E-02 | 0.25 | 1.85E-01 |

### References cited

1. Vogelstein B, Lane D, Levine AJ. Surfing the p53 network. Nature. 2000; 408(6810): 307-10.
2. Hainaut P, Hollstein M. p53 and human cancer: the first ten thousand mutations. Adv Cancer Res. 2000; 77: 81-137.
3. Harris SL, Levine AJ. The p53 pathway: positive and negative feedback loops. Oncogene. 2005; 24(17): 2899-908.
4. Oren M. Decision making by p53: life, death and cancer. Cell Death Differ. 2003; 10(4): 431-42.
5. Shangary S, Wang S. Small-molecule inhibitors of the MDM2-p53 protein-protein interaction to reactivate p53 function: a novel approach for cancer therapy. Annu Rev Pharmacol Toxicol. 2009; 49: 223-41.
6. Manfredi JJ. The Mdm2-p53 relationship evolves: Mdm2 swings both ways as an oncogene and a tumor suppressor. Genes Dev. 2010; 24(15): 1580-9.
7. Vassilev LT. MDM2 inhibitors for cancer therapy. Trends Mol Med. 2007; 13(1): 23-31.
8. Kojima K, Konopleva M, Samudio IJ, Shikami M, Cabreira-Hansen M, McQueen T, et al. MDM2 antagonists induce p53-dependent apoptosis in AML: implications for leukemia therapy. Blood. 2005; 106(9): 3150-9.
9. Vassilev LT, Vu BT, Graves B, Carvajal D, Podlaski F, Filipovic Z, et al. In vivo activation of the p53 pathway by small-molecule antagonists of MDM2. Science. 2004; 303(5659): 844-8.
10. Tovar C, Graves B, Packman K, Filipovic Z, Xia BH, Tardell C, et al. MDM2 Small-Molecule Antagonist RG7112 Activates p53 Signaling and Regresses Human Tumors in Preclinical Cancer Models. Cancer Res. 2013; 73(8): 2587-97.
11. Ray-Coquard I, Blay JY, Italiano A, Le Cesne A, Penel N, Zhi J, et al. Effect of the MDM2 antagonist RG7112 on the P53 pathway in patients with MDM2-amplified, well-differentiated or dedifferentiated liposarcoma: an exploratory proof-of-mechanism study. Lancet Oncol. 2012; 13(11): 1133-40.
12. Thompson T, Andreeff M, Studzinski GP, Vassilev LT. 1,25-dihydroxyvitamin D3 enhances the apoptotic activity of MDM2 antagonist nutlin-3a in acute myeloid leukemia cells expressing wild-type p53. Mol Cancer Ther. 2010; 9(5): 1158-68.
13. Genomic and epigenomic landscapes of adult de novo acute myeloid leukemia. N Engl J Med. 2013; 368(22): 2059-74.
14. Chiaretti S, Tavolaro S, Marinelli M, Messina M, Del Giudice I, Mauro FR, et al. Evaluation of TP53 mutations with the AmpliChip p53 research test in chronic lymphocytic leukemia: correlation with clinical outcome and gene expression profiling. Genes Chromosomes Cancer. 2011; 50(4): 263-74.
15. Bolstad BM, Irizarry RA, Astrand M, Speed TP. A comparison of normalization methods for high density oligonucleotide array data based on variance and bias. Bioinformatics. 2003; 19(2): 185-93.
16. van Bokhoven A, Varella-Garcia M, Korch C, Johannes WU, Smith EE, Miller HL, et al. Molecular characterization of human prostate carcinoma cell lines. Prostate. 2003; 57(3): 205-25.
17. Petitjean A, Mathe E, Kato S, Ishioka C, Tavtigian SV, Hainaut P, et al. Impact of mutant p53 functional properties on TP53 mutation patterns and tumor phenotype: lessons from recent developments in the IARC TP53 database. Hum Mutat. 2007; 28(6): 622-9.
18. Ding Q, Zhang Z, Liu JJ, Jiang N, Zhang J, Ross TM, et al. Discovery of RG7388, a Potent and Selective p53-MDM2 Inhibitor in Clinical Development. J Med Chem. 2013.
19. Barretina J, Caponigro G, Stransky N, Venkatesan K, Margolin AA, Kim S, et al. The Cancer Cell Line Encyclopedia enables predictive modelling of anticancer drug sensitivity. Nature. 2012; 483(7391): 603-7.
20. Garnett MJ, Edelman EJ, Heidorn SJ, Greenman CD, Dastur A, Lau KW, et al. Systematic identification of genomic markers of drug sensitivity in cancer cells. Nature. 2012; 483(7391): 570-5.
21. Higgins B, Tovar C, Glenn K, et al. Antitumor activity of the MDM2 antagonist RG7388. 2013; AACR-NCI-EORTC International Conference on Molecular Targets and Cancer Therapeutics: Abstr B55

## Claims

1. A method to predict responsiveness of a patient, with cancer, to a therapy, said method comprising detecting the mRNA expression level of a four-gene signature consisting of MDM2, XPC, BBC3 and CDKN2A and using that level as a biomarker for predicting the patient's response to a compound, wherein the compound is an inhibitor of the MDM2-p53 interaction, and wherein the response is a response to cancer therapy in a patient and the biomarker is measured *in vitro* in a sample or samples taken from the patient.

2. The method according to claim 1, said method comprising:
a) measuring the mRNA expression level of MDM2, XPC, BBC3 and CDKN2A according to claim 1 in the sample;
b) comparing the level from the patient to standard values from a patient with the same cancer.

3. The method according to any one of claims 1 or 2, wherein the compound is 4-{[2R,3S,4R,5S)-4-(4-Cholor-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid of the formula

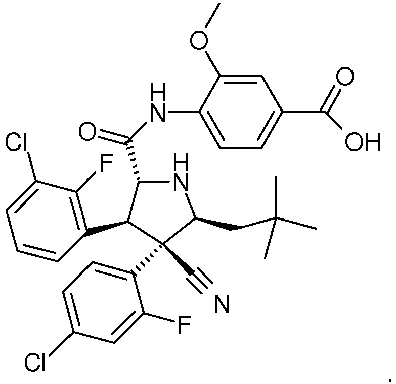

4. The method according to any one of claims 1 or 2, wherein the compound is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl] -amino} -3-methoxy-benzoic acid 1-mPEG-carbonyloxy-ethyl ester (mPEG, average MW, -2000) of the formula:

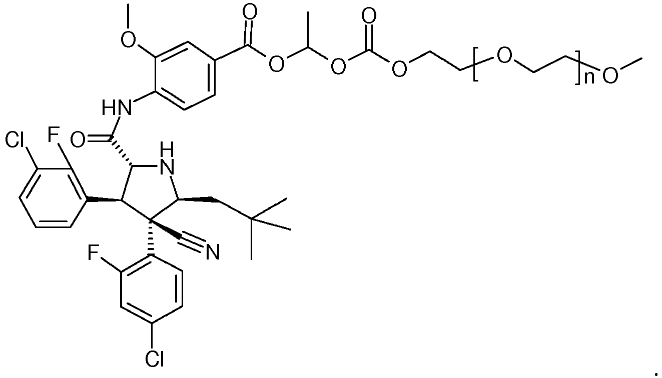

5. The method according to any one of claims 1 or 2, wherein the compound is

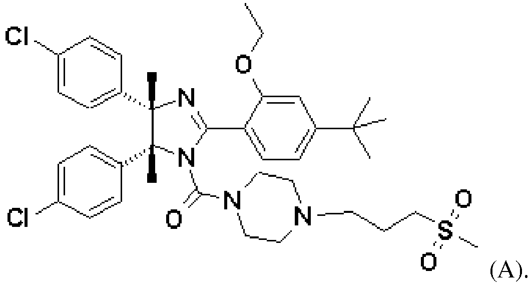

6. The method according to claim 1, **characterized in that** the four-gene signature is composed of up-regulations of three genes including MDM2, XPC, BBC3 and down-regulation of CDKN2A.

7. The method according to claim 1, wherein the cancer is selected from the group consisting of breast cancer, prostate cancer, cervical cancer, ovarian cancer, gastric cancer, colorectal cancer, pancreatic cancer, liver cancer, brain cancer, neuroendocrine cancer, lung cancer, kidney cancer, hematological malignancies, melanoma and sarcomas.

8. The method according to claim 7, wherein the cancer is acute myeloid leukemia (AML).

9. The method according to claim 1, wherein higher MDM2 mRNA expression levels in a sample or samples
i) relative to a standard value or set of standard values from a patient with the same cancer; or
ii) taken after treatment initiation and compared to a sample or samples taken from the same patient before treatment initiation; or
iii) relative to a standard value or set of standard values from normal cells or tissues;
are predictive of sensitivity to treatment with a compound which acts as inhibitor of the MDM2-p53 interaction.

10. An *in vitro* method for monitoring efficacy of therapy comprising an MDM2 inhibitor, in a patient suffering from cancer, the method comprising
a) measuring the mRNA expression level of MDM2, XPC, BBC3 and CDKN2A in a sample obtained from that patient prior to treatment;
b) using the expression levels obtained in a) to calculate a value designated as the patient's signature score prior to treatment;
c) repeating step a) and b) after start of treatment with said MDM2 inhibitor; and
d) comparing the signature scores obtained after start of treatment with those obtained prior to treatment, whereas higher signature scores after treatment indicate a response of the patient to the treatment, and thus a recommendation to continue the treatment.

11. The method of claim 10, wherein the MDM2 inhibitor is the compound used in the methods according to claims 4, 5 or 6.

## Patentansprüche

1. Verfahren zur Vorhersage der Ansprechbarkeit eines Patienten mit Krebs auf eine Therapie, wobei das Verfahren das Nachweisen des mRNA-Expressionsniveaus einer vier-Gensignatur, bestehend aus MDM2, XPC, BBC3 und CDKN2A, und das Verwenden dieses Niveaus als Biomarker zum Vorhersagen der Reaktion eines Patienten auf eine Verbindung umfasst, wobei die Verbindung ein Hemmer der MDM2-p53-Wechselwirkung ist, und wobei die Reaktion eine Reaktion auf die Krebstherapie in einem Patienten ist und der Biomarker *in vitro* in einer dem Patienten entnommenen Probe oder Proben gemessen wird.

2. Verfahren nach Anspruch 1, wobei das Verfahren Folgendes umfasst:
a) Messen des mRNA-Expressionsniveaus von MDM2, XPC, BBC3 und CDKN2A nach Anspruch 1 in der Probe;
b) Vergleichen des Niveaus des Patienten mit Standardwerten eines Patienten mit dem gleichen Krebs.

3. Verfahren nach einem der Ansprüche 1 oder 2, wobei die Verbindung 4-{[2R,3S,4R,5S)-4-(4-Chlor-2-fluorphenyl)-3-(3-chlor-2-fluorphenyl)-4-cyano-5-(2,2-dimethylpropyl)-pyrrolidin-2-carbonyl]-amino}-3-methoxybenzoesäure der Formel 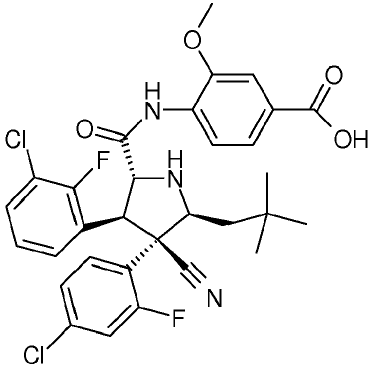 ist.

4. Verfahren nach einem der Ansprüche 1 oder 2, wobei die Verbindung 4-{[(2R,3S,4R,5S)-3-(3-Chlor-2-fluorphenyl)-4-(4-chlor-2-fluorphenyl)-4-cyano-5-(2,2-dimethylpropyl)-pyrrolidin-2-carbonyl]-amino}-3-methoxybenzoesäure-1-mPEG-carbonyloxyethylester (mPEG, durchschnittliches MW, -2000) der Formel: 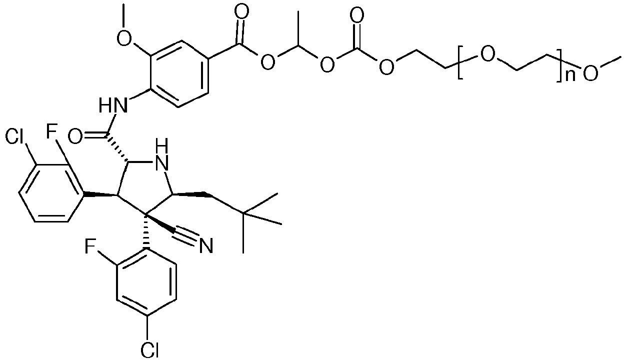 ist.

5. Verfahren nach einem der Ansprüche 1 oder 2, wobei die Verbindung 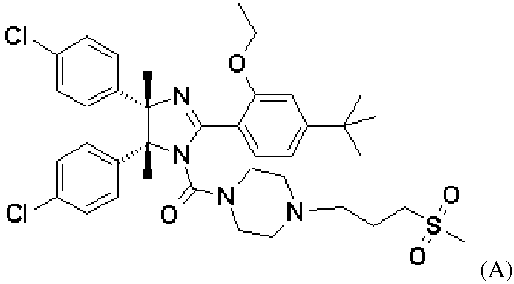 ist.

6. Verfahren nach Anspruch 1, **dadurch gekennzeichnet, dass** die vier-Gensignatur aus Hochregulierungen dreier Gene, einschließlich MDM2, XPC, BBC3, und Herunterregulierung von CDKN2A besteht.

7. Verfahren nach Anspruch 1, wobei der Krebs ausgewählt ist aus der Gruppe bestehend aus Brustkrebs, Prostatakrebs, Gebärmutterhalskrebs, Eierstockkrebs, Magenkrebs, Dickdarmkrebs, Bauchspeicheldrüsenkrebs, Leberkrebs, Hirnkrebs, neuroendokrinem Krebs, Lungenkrebs, Nierenkrebs, hämatologischen Malignitäten, Melanom und Sarkomen.

8. Verfahren nach Anspruch 7, wobei der Krebs akute myeloische Leukämie (AML) ist.

9. Verfahren nach Anspruch 1, wobei höhere MDM2-mRNA-Expressionsniveaus in einer Probe oder Proben
i) in Bezug auf einen Standardwert oder Satz aus Standardwerten eines Patienten mit dem gleichen Krebs; oder
ii) nach dem Behandlungsanfang entnommen und verglichen mit einer vor dem Behandlungsanfang entnommenen Probe oder Proben desselben Patienten; oder
iii) in Bezug auf einen Standardwert oder Satz aus Standardwerten normaler Zellen oder Gewebe;
für Empfindlichkeit gegenüber der Behandlung mit einer Verbindung, die als Hemmer der MDM2-p53-Wechselwirkung agiert, voraussagend sind.

10. *In-vitro-Verfahren* zur Überwachung der Wirksamkeit der Therapie, umfassend einen MDM2-Hemmer, in einem Patienten, der an Krebs leidet, wobei das Verfahren Folgendes umfasst
a) Messen des mRNA-Expressionsniveaus von MDM2, XPC, BBC3 und CDKN2A in einer Probe, die vor der Behandlung vom Patienten erhalten wurde;
b) Verwenden der in a) erhaltenen Expressionsniveaus, um einen Wert zu berechnen, der als Signaturzahl des Patienten vor der Behandlung bezeichnet wird;
c) Wiederholen von Schritt a) und b) nach dem Beginn der Behandlung mit dem MDM2-Hemmer; und
d) Vergleichen der nach dem Beginn der Behandlung erhaltenen Signaturzahlen mit denen, die vor der Behandlung erhalten wurden, während höhere Signaturzahlen nach der Behandlung auf eine Reaktion des Patienten auf die Behandlung und somit auf eine Empfehlung, mit der Behandlung fortzufahren, hinweisen.

11. Verfahren nach Anspruch 10, wobei der MDM2-Hemmer die in den Verfahren nach den Ansprüchen 4, 5 oder 6 verwendete Verbindung ist.

## Revendications

1. Procédé de prédiction de la réponse d'un patient atteint d'un cancer à un traitement, ledit procédé comprenant la détection du niveau d'expression des ARNm d'une signature de quatre gènes constituée de MDM2, XPC, BBC3 et CDKN2A et l'utilisation de ce niveau comme un biomarqueur pour prédire la réponse du patient à un composé, le composé étant un inhibiteur de l'interaction MDM2-p53, et la réponse étant une réponse au traitement anticancéreux chez un patient, et le biomarqueur étant mesuré *in vitro* dans un échantillon ou des échantillons prélevés sur le patient.

2. Procédé selon la revendication 1, ledit procédé comprenant :
a) la mesure du niveau d'expression des ARNm de MDM2, XPC, BBC3 et CDKN2A selon la revendication 1 dans l'échantillon ;
b) la comparaison du niveau du patient à des valeurs de référence d'un patient ayant le même cancer.

3. Procédé selon l'une quelconque des revendications 1 ou 2, dans lequel le composé est l'acide 4-{[2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque de formule

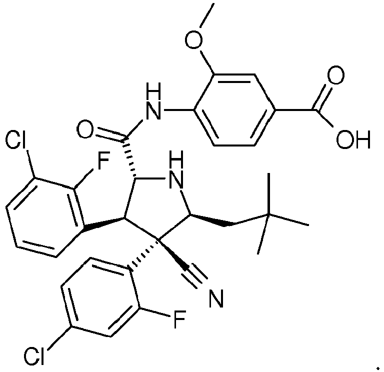

4. Procédé selon l'une quelconque des revendications 1 ou 2, dans lequel le composé est l'ester 1-mPEG-carbonyloxy-éthylique de l'acide 4-{[(2R,3S,4R,5S)-3-(3-chloro-2-fluoro-phényl)-4-(4-chloro-2-fluoro-phényl)-4-cyano-5-(2,2-diméthyl-propyl)-pyrrolidine-2-carbonyl]-amino}-3-méthoxy-benzoïque (mPEG, PM moyen, -2000) de formule :

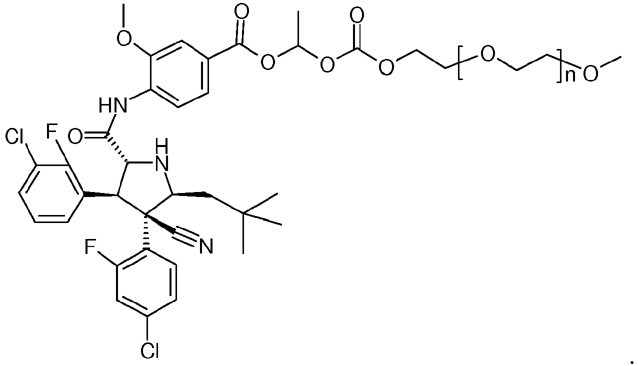

5. Procédé selon l'une quelconque des revendications 1 ou 2, dans lequel le composé est

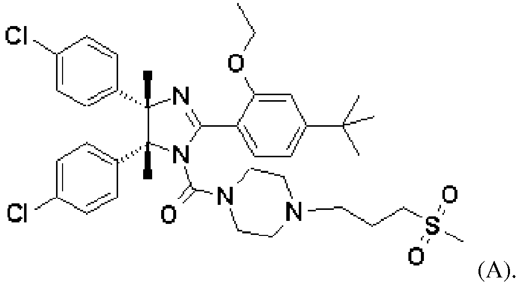

6. Procédé selon la revendication 1, **caractérisé en ce que** la signature de quatre gènes est composée des régulations à la hausse de trois gènes comprenant MDM2, XPC, BBC3 et de la régulation à la baisse de CDKN2A.

7. Procédé selon la revendication 1, dans lequel le cancer est choisi parmi le groupe constitué du cancer du sein, du cancer de la prostate, du cancer du col de l'utérus, du cancer de l'ovaire, du cancer de l'estomac, du cancer colorectal, du cancer du pancréas, du cancer du foie, du cancer du cerveau, du cancer neuroendocrinien, du cancer du poumon, du cancer du rein, des hémopathies malignes, du mélanome et des sarcomes.

8. Procédé selon la revendication 7, dans lequel le cancer est la leucémie myéloïde aiguë (LMA).

9. Procédé selon la revendication 1, dans lequel des niveaux d'expression plus élevés d'ARNm de MDM2 dans un échantillon ou dans des échantillons
i) par rapport à une valeur de référence ou une série de valeurs de référence d'un patient ayant le même cancer ; ou
ii) relevés après l'initiation du traitement et comparés à un échantillon ou des échantillons prélevés sur le même patient avant l'initiation du traitement ; ou
iii) par rapport à une valeur de référence ou une série de valeurs de référence de cellules ou tissus normaux ;
sont prédictifs de la sensibilité au traitement avec un composé qui agit comme inhibiteur de l'interaction MDM2-p53.

10. Procédé *in vitro* de surveillance de l'efficacité d'un traitement comprenant un inhibiteur de MDM2 sur un patient souffrant du cancer, le procédé comprenant
a) la mesure du niveau d'expression des ARNm de MDM2, XPC, BBC3 et CDKN2A dans un échantillon prélevé sur ce patient avant le traitement ;
b) l'utilisation des niveaux d'expression obtenus en a) pour calculer une valeur désignée comme le score de signature du patient avant le traitement ;
c) la répétition des étapes a) et b) après le début du traitement par ledit inhibiteur de MDM2 ; et
d) la comparaison des scores de signatures obtenus après le début du traitement avec ceux obtenus avant le traitement, des scores de signatures plus élevés après le traitement indiquant une réponse du patient au traitement, et ainsi une recommandation de poursuivre le traitement.

11. Procédé de la revendication 10, dans lequel l'inhibiteur de MDM2 est le composé utilisé dans les procédés selon les revendications 4, 5 ou 6.
